(19) Europäisches Patentamt / European Patent Office / Office européen des brevets

(11) **EP 1 746 970 B1**

## (12) FASCICULE DE BREVET EUROPEEN

(45) Date de publication et mention
de la délivrance du brevet:
**31.10.2012 Bulletin 2012/44**

(21) Numéro de dépôt: **05762822.4**

(22) Date de dépôt: **17.05.2005**

(51) Int Cl.:
*A61Q 3/02* (2006.01)          *A61K 8/18* (2006.01)
*A61K 8/87* (2006.01)          *A61K 8/02* (2006.01)
*A45D 29/00* (2006.01)

(86) Numéro de dépôt international:
**PCT/FR2005/050337**

(87) Numéro de publication internationale:
**WO 2005/112875 (01.12.2005 Gazette 2005/48)**

(54) **FILM DE VERNIS A ONGLES AQUEUX**

WÄSSRIGER NAGELLACK

AQUEOUS NAIL VARNISH FILM

(84) Etats contractants désignés:
**AT BE BG CH CY CZ DE DK EE ES FI FR GB GR
HU IE IS IT LI LT LU MC NL PL PT RO SE SI SK TR**

(30) Priorité: **19.05.2004 FR 0451006
29.06.2004 US 583485 P**

(43) Date de publication de la demande:
**31.01.2007 Bulletin 2007/05**

(73) Titulaire: **L'Oréal
75008 Paris (FR)**

(72) Inventeur: **ILEKTI, Philippe
F-94700 MAISONS ALFORT (FR)**

(74) Mandataire: **Le Coupanec, Pascale A.M.P.
Cabinet Nony
3, rue de Penthièvre
75008 Paris (FR)**

(56) Documents cités:
| | |
|---|---|
| **EP-A1- 1 040 814** | **EP-A1- 1 192 930** |
| **WO-A1-97/24103** | **DE-A1- 4 140 888** |
| **FR-A1- 2 803 743** | **GB-A- 1 280 631** |
| **US-A- 5 601 808** | **US-A- 5 747 018** |
| **US-A- 6 123 931** | **US-A- 6 126 929** |
| **US-A1- 2004 000 317** | |

• **DATABASE WPI Section Ch, Week 198827
Derwent Publications Ltd., London, GB; Class
A14, AN 1988-186214 XP002316198 & JP 63
122771 A (SUNSTAR KK) 26 mai 1988 (1988-05-26)**

**Description**

La présente invention concerne un article souple destiné à être appliqué sur les faux ongles ou les ongles pour leur maquillage et/ou leur soin.

Classiquement, le maquillage des ongles ou des faux ongles est réalisé à l'aide de compositions liquides de maquillage, encore communément appelées vernis à ongles. Ce vernis à ongles est appliqué généralement sous la forme de couches superposées à la surface de l'ongle à maquiller, en respectant une étape de séchage intermédiaire entre chaque couche de vernis appliqué. En fait, ce mode de maquillage ne s'avère pas totalement satisfaisant.

Tout d'abord, son application nécessite un certain temps. D'autre part, ce type de maquillage implique d'être répété à bref délai compte tenu de sa tenue insuffisante. En effet, très rapidement, généralement au terme de trois à cinq jours, le vernis appliqué s'écaille et sa brillance diminue. Il est alors nécessaire de procéder à une étape de démaquillage et une nouvelle opération de maquillage.

Par ailleurs, les vernis à ongles classiques impliquent généralement la mise en oeuvre de solvants volatils qui génèrent, lors de l'application, une odeur inconfortable.

Plusieurs alternatives ont déjà été proposées pour tenter de surmonter, au moins en partie, les inconvénients précités. Ainsi, des produits de maquillage des ongles ont été proposés sous la forme de kit de deux compositions liquides de vernis à ongles. Toutefois, l'amélioration de la tenue est dans ce cas acquise au détriment des conditions d'application qui multiplient par deux le nombre de couches à appliquer.

Une autre alternative a consisté à développer des compositions de vernis à ongles à base de dispersion de polymères en phase aqueuse, et donc satisfaisantes en terme olfactif. Malheureusement, les vernis correspondants s'avèrent présenter une tenue insuffisante dans le temps.

FR-A1-2803743 décrit des vernis à ongles comprenant des compositions cosmétiques comprenant des dispersions aqueuses de polymères filmogènes de structure particulières en particules.

US5747018 décrit une composition cosmétique pour le soin des ongles, c'est-à-dire un vernis liquide.

La présente invention vise précisément à proposer un mode de maquillage et/ou de soin des ongles ou des faux ongles permettant précisément, par opposition aux formulations liquides classiques de type vernis à ongles, d'être d'application aisée et rapide pour l'utilisatrice, doté d'une tenue dans le temps significativement améliorée et une teneur nettement réduite en solvant(s) organique(s).

La présente invention propose ainsi un article de maquillage et/ou de soins des ongles ou des faux ongles qui se présente sous la forme d'un film adhésif réalisé à partir d'une dispersion de particules d'au moins un polymère filmogène en phase aqueuse comme décrit à la revendication 1.

La présente invention concerne donc selon un premier aspect, un article souple de maquillage et/ou de soin des ongles et/ou faux ongles comprenant au moins :

- une couche adhésive, et
- au moins un film polymérique obtenu par évaporation de la phase aqueuse d'une dispersion aqueuse de particules d'au moins un polymère filmogène.

Selon une variante de l'invention, l'article comprend en outre entre la couche adhésive et le film polymérique, au moins un film de vernis coloré.

Ce film de vernis est obtenu par réticulation ou évaporation de la phase solvant organique ou aqueuse d'une solution ou dispersion d'au moins un polymère filmogène.

Selon une autre variante de l'invention, le film polymérique est transparent.

Tel qu'utilisé ici, le terme « transparent » signifie que le revêtement a un index HAZEBYK de moins de 5 tel que mesuré avec un brillancemètre de type KYKHAZEGLOSS.

Selon un second aspect, la présente invention concerne un procédé de préparation d'un article souple de maquillage et/ou de soins des ongles, comprenant au moins les étapes consistant à superposer sur un support amovible:

a) au moins une couche d'une composition à base d'au moins un matériau adhésif, et

b) au moins une couche d'une dispersion aqueuse de particules d'au moins un polymère filmogène, l'évaporation de la phase aqueuse étant réalisée consécutivement au dépôt de celle-ci de manière à obtenir un film polymérique.

Selon une première variante de l'invention, le procédé comprend au moins les étapes consistant à :

a) déposer sur un support amovible au moins une couche d'un matériau adhésif,
b) déposer sur ladite couche adhésive au moins une couche d'une dispersion aqueuse de particules d'au moins un polymère filmogène, et
c) évaporer ladite dispersion de manière à obtenir un film polymérique.

Selon une seconde variante de l'invention, le procédé comprend au moins les étapes consistant à :

a) déposer sur un support amovible au moins une couche d'une dispersion aqueuse de particules d'au moins un polymère filmogène,

b) évaporer ladite dispersion de manière à obtenir un film polymérique,

c) déposer sur ledit film obtenu en b) au moins une couche d'un matériau adhésif,

d) si nécessaire sécher au moins partiellement l'article ainsi obtenu,

e) recouvrir la couche adhésive obtenue en c) par un support amovible, et

f) récupérer ledit article par pelage du film polymérique du support en a).

Selon ces deux variantes, l'étape d'évaporation peut être avantageusement modulée pour obtenir un état seulement partiellement sec dudit film polymérique.

Selon une autre variante de l'invention, ledit procédé comprend en outre une étape consistant à former entre la couche adhésive et le film polymérique, un film de vernis coloré.

Selon un troisième aspect, la présente invention concerne un produit de maquillage et/ou de soin des ongles et/ou des faux ongles comprenant dans un conditionnement, sensiblement étanche à l'air, au moins un article conforme à l'invention, le conditionnement étant tel que l'article s'y trouve conservé sous une forme partiellement sèche.

Au sens de la présente invention, le terme partiellement sec entend qualifier le fait que l'article obtenu après formation du film réticulé n'est pas totalement exempt du solvant résiduel. En particulier, il possède une teneur en matière sèche inférieure à 80 %, en particulier inférieure à 75 % et plus particulièrement inférieure à 70 % en poids par rapport à son poids total.

Selon un mode de réalisation particulier, ce conditionnement comporte un réservoir, comme par exemple une poche, souple ou non, apte à contenir de manière étanche un produit pour préserver ledit article d'un séchage total et prématuré avant son utilisation.

Selon un quatrième aspect, la présente invention concerne un procédé de préparation d'un produit tel que défini ci-dessus comprenant les étapes consistant à superposer sur un support amovible :

- au moins une couche d'une composition à base d'au moins un matériau adhésif,
- au moins une couche d'une dispersion aqueuse de particules d'au moins un polymère filmogène, l'évaporation de la phase aqueuse étant réalisée consécutivement au dépôt de ladite composition et contrôlée de manière à obtenir un article partiellement sec, et
- le conditionnement dudit article à un état partiellement sec au sein d'un conditionnement sensiblement étanche à l'air.

Selon ce mode de réalisation, l'article n'acquiert un aspect totalement sec, et donc sa forme définitive qu'après application sur l'ongle, par simple exposition à l'air ambiant.

Selon un cinquième aspect, la présente invention concerne un procédé de maquillage des ongles utilisant un article tel que défini précédemment, comprenant le fait d'appliquer la face adhésive de l'article sur un ongle naturel ou synthétique, et éventuellement le fait d'exercer une pression sur l'article afin de le faire adhérer sur l'ongle.

Les inventeurs ont ainsi constaté qu'il est possible d'obtenir des articles souples de maquillage et/ou de soin des ongles et conformes aux exigences précédentes, sous réserve que cet article possède un film de vernis obtenu par évaporation d'une dispersion aqueuse de particules d'au moins un polymère filmogène.

L'article conforme à l'invention présente avantageusement une longue tenue, en particulier d'au moins cinq jours. Il est en outre résistant à l'eau, aux frottements et aux chocs, ne présentant pas d'usure ni d'écaillage. De plus, l'article conforme à l'invention, présente une facilité d'application particulièrement satisfaisante.

Au sens de la présente invention, le terme « souple » qualifie une flexibilité suffisante de l'article selon l'invention. Plus précisément, cet article se présente sous la forme d'un film apte à se prêter à des déformations mécaniques de type étirement pour l'ajuster à la surface d'un ongle. Cette déformabilité est notamment caractérisée par le paramètre de déformation à la ruptures $\varepsilon_r$ discuté ci-après. A ce titre, l'article selon l'invention se différencie d'un faux ongle qui se caractérise par une rigidité incompatible avec une telle déformation mécanique.

Une autre différence entre l'article conforme à l'invention et un faux ongle, réside dans la sensibilité de cet article vis-à-vis des solvants organiques polaires du type acétone, ester et/ou alcool court. Ainsi, le film polymérique figurant sur la face externe de l'article selon l'invention, c'est-à-dire non adhérente à l'ongle, possède une aptitude à gonfler, et qui notamment se traduit par une augmentation de son poids lorsqu'il est mis en contact avec l'un de ces solvants. Un faux ongle est totalement dénué d'une telle sensibilité. Cette aptitude à gonfler, manifestée par l'article selon l'invention, est précisément avantageuse pour son élimination lorsque celui-ci est appliqué en surface d'un ongle ou d'un faux ongle. En effet, l'article selon l'invention peut être aisément éliminé par simple démaquillage à l'aide d'un dissolvant classique, par opposition à un faux ongle qui se retire.

**ARTICLE SELON L'INVENTION**

L'article selon l'invention se caractérise généralement à l'état sec par un extrait sec élevé. En effet, la quantité de matière sèche est supérieure à 80 %, en particulier supérieure à 85 % et plus particulièrement à 90 % en poids par rapport au poids total du film. Autrement dit la quantité de solvant volatil est inférieure à 20 %, en particulier inférieure à 15 % et plus particulièrement inférieure à 10 % en poids par rapport au poids total du film.

Toutefois, selon un autre mode de réalisation privilégié, l'article selon l'invention peut avantageusement se présenter sous une forme partiellement sèche. Dans ce cas particulier, l'article est conditionné dans un conditionnement de type réservoir comme par exemple une poche, souple ou non, suffisamment étanche pour lui préserver cet aspect partiellement sec. Avantageusement, ce conditionnement est imperméable à l'air et/ou aux solvants. Ce n'est qu'au moment de son utilisation et par conséquent lors de sa mise en contact avec l'air, que l'article sèche totalement pour acquérir la teneur en matière sèche précisée précédemment.

Dans un produit selon l'invention, l'article selon l'invention possède avantageusement une teneur en matière sèche inférieure à 80 %, notamment inférieure à 75 % et plus particulièrement inférieure à 70 % en poids par rapport à son poids total. Ledit article peut par ailleurs posséder une teneur en matière sèche supérieure à 60 %, notamment supérieure à 65 % en poids par rapport à son poids total. Comme précisé précédemment, l'article partiellement sec, extrait du conditionnement et exposé à l'air ambiant, acquiert un état sec tel que défini précédemment dans un délai de 24 h.

De préférence, la quantité de matière sèche, communément appelée « extrait sec » des articles selon l'invention, est mesurée par échauffement de l'échantillon considéré par des rayons infrarouges de 2 $\mu$m à 3,5 $\mu$m de longueur d'onde. Les substances contenues dans lesdits articles qui possèdent une pression de vapeur élevée, s'évaporent sous l'effet de ce rayonnement. La mesure de la perte de poids de l'échantillon permet de déterminer « l'extrait sec » de l'article. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant.

On place environ 10 g d'un échantillon d'article sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante :

$$\text{Extrait Sec} = 100 \text{ x (masse sèche / masse humide).}$$

**Reprise à l'eau**

L'article selon l'invention peut être caractérisé à l'état sec par une reprise à l'eau portée à 25 °C inférieure ou égale à 20 %, notamment inférieure ou égale à 16 %, et en particulier inférieure à 10 %.

Selon la présente demande, on entend par "reprise à l'eau", le pourcentage d'eau absorbé par l'article après 60 minutes d'immersion dans l'eau, à 25 °C (température ambiante). La reprise à l'eau est mesurée pour des morceaux d'environ 1 cm$^2$ découpés dans l'article sec. Ils sont pesés (mesure de la masse M1) puis immergés dans l'eau pendant 60 minutes ; après immersion, le morceau de film est essuyé pour éliminer l'excédent d'eau en surface puis pesé (mesure de la masse M2). La différence M2 - M1 correspond à la quantité d'eau absorbée par le film.

La reprise à l'eau est égale à [(M2 - M1) / M1] x 100 et est exprimée en pourcentage de poids par rapport au poids du film.

**Module de conservation E'**

Par ailleurs, l'article selon l'invention est avantageusement un film ayant un module de conservation E' supérieur ou égal à 1 MPa, notamment allant de 1 MPa à 5000 MPa, en particulier supérieur ou égal à 5 MPa, notamment allant de 5 à 1000 MPa, et plus particulièrement supérieur ou égal à 10 MPa par exemple allant de 10 à 500 MPa à une température de 30 °C et une fréquence de 0,1 Hz.

La mesure du module de conservation est effectuée par DMTA (Dynamical and Mechanical Temperature Analysis ou Analyse dynamique et mécanique en température).

On effectue des essais de viscoélasticimétrie avec un appareil DMTA de Polymer TA Instruments (modèle DMA2980), sur un échantillon d'article. On découpe (par exemple à l'emporte-pièce) les éprouvettes. Celles-ci ont typiquement une épaisseur d'environ 150 $\mu$m, une largeur de 5 à 10 mm et une longueur utile d'environ 10 à 15 mm.

Les mesures sont effectuées à une température constante de 30 °C.

L'échantillon est sollicité en traction et en petites déformations (on lui impose par exemple un déplacement sinusoïdal de $\pm$ 8 $\mu$m) lors d'un balayage en fréquence, la fréquence allant de 0,1 à 20 Hz. On travaille ainsi dans le domaine linéaire, sous de faibles niveaux de déformation.

Ces mesures permettent de déterminer le module complexe E* = E' + iE" du film de composition testé, E' étant le module de conservation et E" le module dit de perte.

## Déformation et Energie à la rupture

Avantageusement, les articles selon l'invention, possèdent une déformation à la rupture $\varepsilon_r$ supérieure ou égale à 5 %, notamment allant de 5 à 500 %, de préférence supérieure ou égale à 15 %, notamment allant de 15 à 400 % et/ou une énergie à rupture par unité de volume $W_r$ supérieure ou égale à 0,2 J/cm$^3$, notamment allant de 0,2 à 1.00 J/cm$^3$, de préférence supérieure à 1 J/cm$^3$, notamment allant de 1 à 50 J/cm$^3$.

La déformation à la rupture et l'énergie à rupture par unité de volume sont déterminées par des essais de traction effectués sur un film d'environ 200 $\mu$m d'épaisseur.

Pour effectuer ces essais, l'article est découpé en éprouvettes haltères de longueur utile 33 $\pm$ 1 mm et de largeur utile 6 mm. La section (S) de l'éprouvette est alors définie comme : S = largeur x épaisseur (cm$^2$); cette section sera utilisée pour le calcul de la contrainte.

Les essais sont réalisés, par exemple, sur un appareil de traction commercialisé sous l'appellation Lloyd® LR5K. Les mesures sont réalisées à température ambiante (20 °C).

Les éprouvettes sont étirées à une vitesse de déplacement de 33 mm/min, correspondant à une vitesse de 100 % d'allongement par minute.

On impose donc une vitesse de déplacement et on mesure simultanément l'allongement $\Delta$L de l'éprouvette et la force F nécessaire pour imposer cet allongement. C'est à partir de ces données $\Delta$L et F que l'on détermine les paramètres contraintes $\sigma$ et déformation $\varepsilon$.

Il est ainsi obtenu une courbe contrainte $\sigma$ = (F/S) en fonction de la déformation $\varepsilon$ = ($\Delta$L/L$_o$) x 100, l'essai étant conduit jusqu'à rupture de l'éprouvette, L$_o$ étant la longueur initiale de l'éprouvette.

La déformation à la rupture $\varepsilon_r$ est la déformation maximale de l'échantillon avant le point de rupture (en %).

L'énergie à rupture par unité de volume $W_r$ en J/cm$^3$ est définie comme la surface sous cette courbe contrainte/déformation telle que :

$$Wr = \int_{0}^{\varepsilon_r} \sigma \, \varepsilon.d\varepsilon$$

## DISPERSION AQUEUSE DE PARTICULES DE POLYMERE(S) FILMOGENE(S)

Comme indiqué précédemment, l'article conforme à l'invention comporte un film obtenu par évaporation d'au moins une dispersion aqueuse de particules d'au moins un polymère filmogène. Ces particules solides peuvent être de nature anionique, cationique ou neutre.

Dans la présente invention, on entend par « aqueux », un milieu liquide à base d'eau et/ou de solvants hydrophiles. Ce milieu liquide aqueux peut être constitué essentiellement d'eau. Il peut également comprendre un mélange d'eau et de solvant(s) organique(s) miscible(s) à l'eau (miscibilité dans l'eau supérieure à 50 % en poids à 25 °C) comme les monoalcools inférieure ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$.

Dans la présente demande, on entend par "polymère filmogène", un polymère apte à former à lui seul ou en présence d'un agent auxiliaire de filmification, un film continu sur un support, à une température allant de 20 °C à 150 °C.

Conformément à la présente invention, le polymère filmogène est présent sous la forme de particules en dispersion aqueuse, connue généralement sous le nom de latex ou pseudolatex. Les techniques de préparation de ces dispersions sont bien connues de l'homme du métier.

Une dispersion convenant à l'invention, peut comprendre un ou plusieurs types de particules, ces particules pouvant varier par leur taille, par leur structure et/ou par leur nature chimique.

La taille des particules de polymères en dispersion aqueuse peut varier de 5 à 500 nm, et est en particulier de 10 à 150 nm. On peut toutefois utiliser des particules de taille allant jusqu'à 1 micron.

La taille des particules peut être mesurée par exemple avec un appareil de type Brookhaven BI-90 par la technique de diffusion de la lumière, ou avec un granulomètre de type Malvern mastersizer 2000, ou bien encore par microscopie électronique.

De manière générale, la dispersion aqueuse utilisée pour réaliser un article conforme à l'invention comprend de 0,5 à 60 % en poids, et en particulier de 1 à 50 % en poids total de matière sèche de polymère filmogène, par rapport au poids total de la dispersion.

Selon un mode de réalisation particulier, l'article de l'invention est un film multicouche réalisé en plusieurs étapes à partir de différentes dispersions aqueuses de polymère filmogène.

Plus précisément, il peut s'agir d'un film multicouche réalisé par superposition d'au moins deux voire plus de couches obtenues respectivement par évaporation de la phase aqueuse de dispersions de particules de polymère(s) filmogène(s) de natures différentes. Dans un tel un mode de réalisation particulier, on peut utiliser une première dispersion aqueuse d'au moins un polymère filmogène ayant au moins une, notamment ayant une, température de transition vitreuse Tg1 inférieure ou égale à 20 °C, notamment allant de -120 °C à 20 °C, et en particulier inférieure à 10 °C, notamment allant de -120 °C à 0 °C, et plus particulièrement allant de -70 °C à -30 °C. La dispersion est déposée sur un support, puis est séchée à une température de 50 à 150 °C. On utilise ensuite une deuxième dispersion aqueuse qu'on dépose sur cette première couche, deuxième dispersion aqueuse d'au moins un polymère filmogène ayant au moins une température de transition vitreuse Tg2 supérieure ou égale à 30 °C, notamment allant de 30 °C à 200 °C, et avantageusement supérieure ou égale à 50 °C, notamment allant de 50 °C à 200 °C, et en particulier supérieure ou égale à 80 °C, notamment allant de 80 °C à 180 °C. L'ensemble est ensuite séché généralement partiellement à une température de 50 °C à 150 °C, en particulier à une température supérieure à 100 °C.

Parmi les polymères filmogènes utilisables dans la composition de la présente invention, on peut citer les polymères synthétiques, de type radicalaire ou de type polycondensat, les polymères d'origine naturelle et leurs mélanges. D'une manière générale ces polymères peuvent être des polymères statistiques, des copolymères blocs de type A-B, multi blocs A- B-A ou encore ABCD..., voire des polymères greffés.

### Polymère filmogène radicalaire,

Par « polymère radicalaire », on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des homopolymères ou des copolymères, acryliques et /ou vinyliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomères à insaturation éthylénique ayant au moins un groupement acide ou monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise en particulier l'acide (méth) acrylique et l'acide crotonique, et plus particulièrement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{20}$, plus particulièrement en $C_1$-$C_8$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique sont en particulier des (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acide, on peut par exemple citer les (méth)acrylamides, et notamment les N-alkyl (méth)acrylamides, en particulier d'alkyle en $C_2$-$C_{12}$. Parmi les N-alkyl (méth)acrylamides, on peut citer le N-éthyl acrylamide, le N-t-butyl acrylamide et le N-t-octyl acrylamide.

Les polymères filmogènes vinyliques peuvent également résulter de l'homopolymérisation ou de la copolymérisation de monomères choisis parmi les esters vinyliques et les monomères styréniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styréniques, on peut citer le styrène de l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère vinylique, on peut également utiliser les polymères acryliques siliconés.

On peut également citer les polymères résultant de la polymérisation radicalaire d'un ou plusieurs monomères radicalaires, à l'intérieur et/ou partiellement à la surface de particules préexistantes d'au moins un polymère choisi parmi le groupe constitué par les polyuréthanes, les polyurées, les polyesters, les polyesteramides et/ou les alkydes. Ces polymères sont généralement appelés « polymères hybrides ».

## Polycondensat

Comme polymère filmogène de type polycondensat, on peut citer les polyuréthannes anioniques, cationiques, non-ioniques ou amphotères, les polyuréthannes-acryliques, les polyuréthannes-polyvinylpyrrolidones, les polyester-polyuréthannes, les polyéther-polyuréthannes, les polyurées, les polyurée/polyuréthannes, les polyuréthannes siliconés, et leurs mélanges.

Le polyuréthanne filmogène peut être, par exemple, un copolymère polyuréthanne, polyurée/uréthanne, ou polyurée, aliphatique, cycloaliphatique ou aromatique, comportant seule ou en mélange au moins une séquence choisie parmi :

- une séquence d'origine polyester aliphatique et/ou cycloaliphatique et/ou aromatique, et/ou,
- une séquence siliconée, ramifiée ou non, par exemple polydiméthylsiloxane ou polyméthylphénylsiloxane, et/ou
- une séquence comportant des groupes fluorés.

Les polyuréthannes filmogènes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

Parmi les polycondensats filmogènes, on peut également citer les polyesters, les polyesters amides, les polyesters à chaîne grasse, les polyamides, et les résines époxyesters.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylgluratique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarbocylique, l'acide 1,4-cyclohexanedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalène-dicarboxylique. Ces monomères acide dicarboxylique peuvent être utilisés seuls ou en combinaison d'au moins deux monomères acide dicarboxylique. Parmi ces monomères, on choisit en particulier l'acide phtalique, l'acide isophtalique et l'acide téréphtalique.

Le diol peut être choisi parmi les diols aliphatiques, alicycliques, aromatiques. On utilise en particulier un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

## Polymère d'origine naturelle

On peut utiliser dans la présente invention des polymères d'origine naturelle, éventuellement modifiés, comme la résine shellac, la gomme de sandaraque, les dammars, les élémis, les copals, les polymères cellulosiques insolubles dans l'eau tels que la nitrocellulose, les esters de cellulose modifiés dont notamment des esters de carboxyalkyl cellulose comme ceux décrits dans la demande de brevet US 2003/185774, et leurs mélanges.

Selon une première variante de l'invention, la dispersion aqueuse de particules de polymère est une dispersion aqueuse de particules de polyester-polyuréthanne et/ou de polyéther-polyuréthanne en particulier anionique.

Le caractère anionique des polyester-polyuréthannes et des polyéther-polyuréthannes utilisés selon l'invention est du à la présence dans leurs motifs constitutifs de groupements à fonction acide carboxylique ou acide sulfonique.

Les particules de polyester-polyuréthanne ou de polyéther-polyuréthanne utilisées selon l'invention sont généralement commercialisées sous forme de dispersions aqueuses.

Avantageusement, les particules présentent une taille allant de 5 à 500 nm, et en particulier allant de 10 à 250 nm.

La teneur en particules desdites dispersions actuellement disponibles sur le marché, va d'environ 20 % à environ 50 % en poids par rapport au poids total de la dispersion.

Parmi les dispersions de polyester-polyuréthanne anionique utilisables dans les vernis aqueux selon l'invention, on peut citer en particulier celles commercialisées sous les dénominations "Sancure 2060®" et "Sancure 815®" par la société SANNCOR.

Parmi les dispersions de particules de polyéther-polyuréthanne anionique utilisables selon l'invention, on peut citer en particulier celles commercialisées sous la dénomination de "Sancure 878®" par la société SANNCOR, et sous la dénomination de "Néorez R 970®" par la société ICI.

Selon un mode de réalisation particulier de l'invention, on peut utiliser un mélange de dispersions commerciales constitué de particules de polyester-polyuréthanne anionique telles que définies ci-dessus et de particules de polyéther-polyuréthanne anionique également définies ci-dessus.

Par exemple, on peut utiliser un mélange constitué de la dispersion commercialisée sous la dénomination de "Sancure 2060®" et de celle commercialisée sous la dénomination de "Sancure 861®" ou un mélange de celle commercialisée sous la dénomination de "Sancure 815®" et de celle commercialisée sous la dénomination de "Sancure 878®", ces dispersions étant commercialisées par la société SANNCOR.

On utilise en particulier des mélanges contenant respectivement 60 % et 70 % de particules de polyester-polyuréthanne, le reste étant constitué par des particules de polyéther-polyuréthanne.

Dans cette première variante particulière de l'invention, la dispersion de particules est mise en oeuvre sous la forme d'une dispersion dont la teneur en particules de polyester-polyuréthanne et/ou de polyéther-polyuréthanne va généralement de 3 à 50 %, et en particulier de 10 à 50 % en poids par rapport au poids total de la dispersion.

Selon une seconde variante de l'invention, la dispersion aqueuse de particules de polymère est une dispersion aqueuse de polymère acrylique.

Ce polymère présente des propriétés de solubilité à 25 °C dans des solvants organiques correspondant aux paramètres moyens de solubilité dD, dP, et dH de HANSEN satisfaisant aux conditions suivantes :

- dD = 17,5
- dP = 7
- dH = 7,6

avec un rayon R allant de 5 à 10, et en particulier de 5 à 6.

La définition des solvants dans l'espace de solubilité tridimensionnel selon HANSEN est décrite dans l'article de C. M. HANSEN : "The three dimensionnal solubility parameters" J. Paint Technol. 39, 105 (1967).

- dD caractérise les forces de dispersion de LONDON issues de la formation de dipôles induits lors des chocs moléculaires.
- dP caractérise les forces d'interaction de DEBYE entre dipôles permanents ainsi que les forces d'interactions de KEESOM entre dipôles induits et dipôles permanents.
- dH caractérise les forces d'interactions spécifiques (type liaison hydrogène, acide/base, donneur/accepteur, etc...).
- les paramètres dD, dP, dH sont exprimés en $(J/cm^3)^{1/2}$.

Le rayon R correspond à la distance séparant, dans l'espace des paramètres de solubilité de Hansen, un solvant organique du point dudit espace correspondant à dD = 17,5 ; dP = 7 ; dH = 7,6, R vérifiant la relation suivante :

$$5 \, J^{1/2}cm^{-3/2} \leq R \leq 10 \, J^{1/2}cm^{-3/2},$$

dans laquelle:

$$R = \sqrt{4(\delta^s_d - 17,5)^2 + (\delta^s_p - 7)^2 + (\delta^s_h - 7,6)^2}$$

et où $\delta^s_d$, $\delta^s_p$, $\delta^b_h$ sont les paramètres de solubilité de Hansen d'un solvant organique pour lequel le polymère acrylique utilisé dans la présente invention présente des propriétés de solubilité. La définition du rayon R est connue de l'ouvrage de Allan F. M. Barton, CRC Handbook of solubility parameters and other cohesion parameters, Second edition, 1991, pages 95 à 109.

Le polymère acrylique peut être un copolymère styrène/acrylate, et notamment un polymère choisi parmi les copolymères issus de la polymérisation d'au moins un monomère styrénique et au moins un monomère (méth)acrylate d'alkyle en $C_1$-$C_{18}$.

Comme monomère styrénique utilisable dans l'invention, on peut citer par exemple le styrène ou l'alpha-méthylstyrène, et en particulier le styrène.

Le monomère de (méth)acrylate d'alkyle en $C_1$-$C_{18}$ est en particulier un (méth)acrylate alkyle en $C_1$-$C_{12}$ et plus particulièrement un (méth)acrylate d'alkyle en $C_1$-$C_{10}$. Le monomère (méth)acrylate d'alkyle en $C_1$-$C_{18}$ peut être choisi parmi l'acrylate de méthyle, le méthacrylate de méthyle, l'acrylate d'éthyle, l'acrylate de propyle, l'acrylate de butyle, le méthacrylate de butyle, l'acrylate d'hexyle, l'acrylate d'octyle, l'acrylate de 2-éthyl hexyle, le (méth)acrylate de lauryle et le (méth)acrylate de stéaryle.

Comme polymère acrylique en dispersion aqueuse, on peut utiliser selon l'invention le copolymère styrène/acrylate commercialisé sous la dénomination "Joncryl SCX-8211®" par la société JOHNSON.

Selon un mode de réalisation particulier de l'invention, la dispersion peut comprendre comme unique polymère en dispersion aqueuse le polymère acrylique défini précédemment.

Dans cette seconde variante particulière de l'invention, la teneur en particules de polymère acrylique en matière sèche, est celle qui est efficace pour former un film, notamment celle allant de 3 % à 60 % en poids, et en particulier de 5 % à 50 % en poids, par rapport au poids total de la dispersion.

Selon une troisième variante de l'invention, la dispersion aqueuse utilisée comprend un mélange d'au moins deux polymères filmogènes sous forme de particules solides distincts par leurs Tg respectifs.

Plus précisément, elle comprend dans un milieu aqueux acceptable :

a) des particules solides dispersées dans le milieu aqueux d'un premier polymère filmogène ayant au moins une température de transition vitreuse Tg1 supérieure ou égale à 30 °C, et
b) des particules solides dispersées dans le milieu aqueux d'un deuxième polymère filmogène ayant au moins une température de transition vitreuse Tg2 inférieure ou égale à 0 °C,

le premier polymère filmogène étant présent en une teneur allant de 50 % à 90 % en poids, par rapport au poids total des premier et deuxième polymères filmogènes.

Cette dispersion résulte généralement d'un mélange de deux dispersions aqueuses de polymère filmogène.

Le premier polymère filmogène a au moins une, notamment a une, température de transition vitreuse Tg1 supérieure ou égale à 30 °C, notamment allant de 30 °C à 200 °C, et avantageusement supérieure ou égale à 50 °C, notamment allant de 50 °C à 200 °C, et en particulier supérieure ou égale à 80 °C, notamment allant de 80 °C à 180 °C.

Le deuxième polymère filmogène a au moins une, notamment a une, température de transition vitreuse Tg2 inférieure ou égale à 0 °C, notamment allant de - 120 °C à 0 °C, et en particulier inférieure à -10 °C, notamment allant de -120 °C à -10 °C, et plus particulièrement allant de -30 °C à -70 °C.

La mesure de la température de transition vitreuse (Tg) d'un polymère est effectuée par DMTA (Dynamical and Mechanical Temperature Analysis ou Analyse dynamique et mécanique de température) comme décrit ci-dessous.

Pour mesurer la température de transition vitreuse (Tg) d'un polymère, on effectue des essais de viscoélasticimétrie avec un appareil DMTA de "Polymer laboratories", sur un échantillon de film. Ce film est préparé par coulage de la dispersion aqueuse de polymère filmogène dans une matrice téflonnée puis séché à 120 °C pendant 24 heures. On obtient alors un film dans lequel on découpe (par exemple à l'emporte-pièce) les éprouvettes. Celles-ci ont typiquement une épaisseur d'environ 150 μm, une largeur de 5 à 10 mm et une longueur utile d'environ 10 à 15 mm. On impose à cet échantillon une sollicitation de traction. L'échantillon subit une force statique de 0,01 N à laquelle se superpose un déplacement sinusoïdal de $\pm$ 8 μm à la fréquence de 1 Hz. On travaille ainsi dans le domaine linéaire, sous de faibles niveaux de déformation. Cette sollicitation de traction est effectuée sur l'échantillon à des températures variant de -150 °C à +200 °C, avec une variation de température de 3 °C par minute.

On mesure alors le module complexe $E^* = E' + iE''$ du polymère testé en fonction de la température.

De ces mesures, on déduit les modules dynamiques E', E" et le pouvoir amortissant : $tg\delta = E''/E'$.

Puis on trace la courbe des valeurs de $tg\delta$ en fonction de la température ; cette courbe présente au moins un pic. La température de transition vitreuse Tg du polymère correspond à la température à laquelle se situe le sommet de ce pic.

Lorsque la courbe présente au moins 2 pics (dans ce cas, le polymère présente au moins 2 Tg), on prend comme valeur de Tg du polymère testé la température pour laquelle la courbe présente un pic de plus forte amplitude (c'est-à-dire correspondant à la plus grande valeur de $tg\delta$; on ne considère dans ce cas que la Tg "majoritaire" comme valeur de Tg du polymère testé).

Dans la présente invention, la température de transition Tg1 correspond à la Tg "majoritaire" (au sens défini précédemment) du premier polymère filmogène lorsque ce dernier présente au moins 2 Tg ; la température de transition vitreuse Tg2 correspond à la Tg "majoritaire" du deuxième polymère filmogène lorsque ce dernier présente au moins 2 Tg.

Le premier polymère filmogène et le deuxième polymère filmogène peuvent être choisis, indépendamment l'un de l'autre, parmi les polymères radicalaires, les polycondensats et les polymères d'origine naturelle tels que définis précédemment ayant les caractéristiques de température de transition vitreuse définies précédemment.

Comme premier polymère filmogène en dispersion aqueuse, on peut utiliser les dispersions aqueuses de polymère vendues sous les dénominations "NeoRez R-989®" par la société AVECIA RESINS, "Avalure® UR405" par la société NOVEON ou "Bayderm Finish DLH®" par la société BAYER.

Comme deuxième polymère filmogène en dispersion aqueuse, on peut utiliser par exemple les dispersions aqueuses de polymère vendues sous les dénominations "Avalure® UR-460" par la société NOVEON ou "Acrilem IC89RT®" par la société ICAP.

Le polymère filmogène de la dispersion aqueuse "Avalure® UR-460" est un polyuréthanne obtenu par la polycondensation de polyoxyde de tétraméthylène, de diisocyanate de tétraméthylxylylène, de diisocyanate d'isophorone et d'acide diméthylol propionique.

Selon un mode de réalisation particulier de cette troisième variante de l'invention, la dispersion aqueuse de particules est une dispersion aqueuse de particules d'au moins un polyuréthanne et d'au moins un polymère radicalaire à groupement carboxylique.

Une dispersion aqueuse comprenant des particules de polyuréthanne et de polymère radicalaire à groupement carboxylique conforme à l'invention peut être préparée par exemple par simple mélange d'une dispersion aqueuse de polyuréthanne et d'une dispersion aqueuse de polymère radicalaire, ou par formation directe d'une dispersion d'un mélange de particules de polyuréthanne et de polymère radicalaire.

La dispersion aqueuse de polyuréthanne peut être, par exemple, une dispersion aqueuse de polyuréthanne anionique, de polyester-polyuréthanne et/ou de polyéther-polyuréthanne, seul ou en mélange, pouvant présenter un taux de matière sèche de 10-50%.

Par exemple, on utilisera dans la présente invention selon cette variante un mélange d'une dispersion de polyuréthanne et d'une dispersion de polymères radicalaires obtenu par mélange puis agitation pendant environ 30 minutes à température ambiante selon les conditions suivantes :

(i) polymères employés :

- radicalaire, Tg = 35 °C
- (appelé polymère 1A) : dispersion d'acrylique styrène "Joncryl 77®" de Johnson, à 46 % de matière sèche,

- radicalaire, Tg = 65 °C
- (appelé polymère 1B) : dispersion d'acrylique styrène "Neocryl XK 63®" de ICI, à 44 % de matière sèche,

- radicalaire, Tg = 110 °C
- (appelé polymère 1C) : dispersion d'acrylique styrène "Joncryl 90®" de Johnson, à 44 % de matière sèche.

- polyuréthanne, Tg = -10 °C (appelé polymère 2) : dispersion de polyuréthanne anionique aliphatique "IW/019.1®" de UCB, à 35 % de matière sèche,

(ii) proportion et nature des ingrédients du mélange :

| Polymère 2 % | Polymère 1 | % | Extrait sec % |
|---|---|---|---|
| 50 | A | 50 | 35 |
| 50 | B | 50 | 35 |
| 50 | C | 50 | 35 |

- extrait sec : taux de matière sèche de la dispersion avant évaporation,
- % : % de dispersion de polyuréthanne ou de dispersion de polymères radicalaires dans le mélange c'est-à-dire avant évaporation.

Les particules du premier et du deuxième polymères filmogènes peuvent avoir une taille moyenne, indépendamment les unes des autres, allant de 10 nm à 500 nm, et notamment allant de 20 nm à 300 nm.

Dans la dispersion utilisée selon la troisième variante de l'invention, le premier polymère filmogène est généralement présent en une teneur allant de 50 % à 90 % en poids, par rapport au poids total des premier et deuxième polymères. Par analogie, le deuxième polymère filmogène est donc présent dans la dispersion en une teneur allant de 10 % à 50 % en poids, par rapport au poids total des premier et deuxième polymères filmogènes.

Selon un mode de réalisation particulier, le premier polymère filmogène peut être présent dans le mélange de polymères en une teneur allant de 70 % à 90 % en poids, par rapport au poids total des premier et deuxième polymères filmogènes, et plus particulièrement allant de 70 % à 85 % en poids. Dans ce cas, le deuxième polymère est présent dans le mélange de polymères filmogènes en une teneur allant respectivement de 10 % à 30 % en poids, par rapport au poids total des premier et deuxième polymères filmogènes, et plus particulièrement de 15 % à 30 % en poids.

Dans cette troisième variante, la teneur totale en particules du premier polymère filmogène et du deuxième polymère filmogène est généralement de 0,1 % à 60 % en poids, par rapport au poids total de la dispersion, en particulier de 1 % à 50 %, et plus particulièrement de 5 % à 40 % en poids

Selon une quatrième variante de l'invention, la dispersion aqueuse utilisée comprend au moins une dispersion aqueuse multiphasée particulière.

Plus précisément, elle comprend dans un milieu aqueux acceptable une dispersion de particules, les particules comprenant au moins une phase souple au moins en partie externe comportant au moins un polymère souple ayant au moins une température de transition vitreuse inférieure ou égal à 60 °C et au moins une phase rigide au moins en partie interne, la phase rigide étant un matériau amorphe ayant au moins une température de transition vitreuse supérieure à 60 °C, le polymère souple étant fixé au moins partiellement par greffage chimique sur la phase rigide.

Les particules selon l'invention, appelées aussi particules multiphasées (ou composites), sont des particules comprenant au moins une phase souple et au moins une phase rigide.

Le polymère souple des particules en dispersion a au moins une température de transition vitreuse inférieure ou égale à 60 °C, notamment allant de - 120 °C à 60 °C, en particulier inférieure ou égale à 45 °C, notamment allant de - 120 °C à 45 °C, et plus particulièrement inférieure ou égale à 30°C, notamment allant de - 120 °C à 30 °C.

Le polymère souple peut être choisi parmi les polymères séquencés et/ou statistiques. Par « polymères séquencés et/ou statistiques », on entend des polymères dont la répartition des monomères sur la chaîne principale ou les chaînons pendants est séquencée (blocs) et/ou statistique.

Le polymère souple peut être choisi parmi les polymères radicalaires, les polycondensats, les polymères siliconés. Le polymère souple peut être choisi parmi les polyacryliques, les polyméthacryliques, les polyamides, les polyuréthannes, les polyoléfines notamment les polyisoprènes, polybutadiènes, polyisobutylènes (PIB), les polyesters, les polyvinyléthers, les polyvinylthioéthers, les polyoxydes, les polysiloxanes et notamment les polydiméthyl siloxanes (PDMS), et leurs associations. Par associations, on entend les copolymères pouvant être formés à partir des monomères conduisant à la formation desdits polymères.

En particulier, le polymère souple peut être choisi parmi les poly(méth)acryliques, les polyuréthanes, les polyoléfines, les polysiloxanes.

Le matériau amorphe de la phase rigide a une température de transition vitreuse supérieure à 60 °C, notamment supérieure à 60 °C et inférieure ou égale à 200 °C, en particulier supérieure ou égale à 70 °C, notamment allant de 70 °C à 200 °C, plus particulièrement allant de 70 °C à 150 °C, voire supérieure ou égale à 90 °C, notamment allant de 90 °C à 150 °C.

Le matériau amorphe de la phase rigide peut être un polymère, notamment un polymère séquencé et/ou statistique. Il peut être un polymère choisi parmi les polyacryliques, les polyméthacryliques comme par exemple les poly acide (méth)acrylique, les poly(méth)acrylamides, les polyvinyliques, les polyvinylesters, les polyoléfines, les polystyrènes, les polyvinylhalogénures comme le polychlorure de vinyle (PVC), les polyvinylnitriles, les polyuréthannes, les polyesters, les polyamides, les polycarbonates, les polysulfones, les polysulfonamides, les polycycliques ayant un cycle carboné dans la chaîne principale comme des polyphénylènes, les polyoxyphénylènes, et leurs associations.

Avantageusement, le matériau amorphe de la phase rigide peut être un polymère choisi parmi les polyacryliques, les polyméthacryliques comme par exemple les poly acide (méth)acrylique, les poly(méth)acrylamides, les polyvinyliques, les polyvinylesters, les polyoléfines, les polystyrènes, les polyvinylhalogénures comme le polychlorure de vinyle (PVC), les polyvinylnitriles, les polyuréthannes, les polyamides, les polyesters.

Selon un mode de réalisation particulier de l'invention, les phases souple et rigide des particules multiphasées peuvent comprendre au moins un polymère radicalaire obtenu par, voire essentiellement par, polymérisation de monomères choisis dans le groupe formé par :

- les esters de l'acide (méth)acrylique, comme les (méth)acrylates d'alkyle, notamment ayant un groupe alkyle en $C_1$-$C_8$,
- les esters vinyliques des acides carboxyliques linéaires ou branchés, tels que l'acétate de vinyle ou le stéarate de vinyle,
- le styrène et ses dérivés, tels que le chloro méthyl styrène, l'alpha méthyl styrène,
- les diènes conjugués, tels que le butadiène ou l'isoprène,
- l'acrylamide, le méthacrylamide et l'acrylonitrile,
- le chlorure de vinyle, et
- l'acide (méth)acrylique.

La sélection de monomères (nature et teneur), qui peut être un seul monomère ou un mélange d'au moins deux monomères, du polymère souple et du matériau amorphe de la phase rigide est déterminée par la température de transition vitreuse que l'on souhaite conférer à chaque polymère.

Les polymères des phases rigide et/ou souple peuvent être réticulés à l'aide de monomères possédant au moins deux doubles liaisons copolymérisables, par exemple choisis parmi :

- les diènes conjugués, tel que le butadiène ou l'isoprène,
- les esters allyliques d'acides carboxyliques alpha béta insaturés tels que l'acrylate d'allyle, le méthacrylate d'allyle,
- les esters allyliques d'acides dicarboxyliques alpha béta insaturés tel que le maléate de diallyle,
- les polyacryliques ou polyméthacryliques comprenant généralement au moins deux insaturations éthyléniques tels que le diméthacrylate d'éthylène glycol, le diméthacrylate de 1,3-butylène glycol, le diacrylate de 1,4-butanediol, ou le tétraacrylate de pentaneérythritol ;
- les polyvinyliques tels que le divinylbenzène ou le trivinyl benzène, et
- les polyallyliques tel que le cyanurate de triallyle.

Le greffage chimique permet, par la formation de liaisons covalentes, de lier la phase rigide et la phase souple des particules multiphasées de manière stable.

Le greffage chimique peut se faire par polymérisation radicalaire séquencée (appelée aussi polymérisation séquencielle) selon les modes opératoires bien connus de l'homme de l'art. La polymérisation séquencée consiste à polymériser dans une première étape les monomères du polymère rigide (polymère formant la phase rigide des particules) puis de poursuivre lors d'une deuxième étape la polymérisation avec les monomères formant le polymère souple (polymère formant la phase souple des particules). De cette façon, les chaînes de polymère de la phase souple sont liées au moins en partie par liaison covalente aux chaînes du polymère de la phase rigide, la liaison covalente résultant de la polymérisation d'un monomère du polymère souple avec un monomère du polymère rigide. Avantageusement, les monomères du polymère de la phase souple externe présentent une affinité plus importante pour le milieu de dispersion que les monomères du polymère de la phase rigide interne.

Le polymère souple peut être greffé sur le polymère rigide par l'intermédiaire de monomère greffant, ce dernier pouvant être un monomère possédant plusieurs doubles liaisons (liaisons éthyléniques), en particulier un monomère possédant deux doubles liaisons éthyléniques. Le monomère greffant peut être un diène conjugué tels que ceux décrits précédemment ou un ester (notamment diester) allylique d'acides dicarboxyliques alpha béta insaturés tels que ceux décrits précédemment (comme par exemple le maléate de diallyle) qui possèdent deux fonctions polymérisables (double liaison éthylénique) de réactivités différentes : l'une des fonctions polymérisables (double liaison éthylénique) du monomère greffant est polymérisée avec le polymère du matériau amorphe de la phase rigide (polymère rigide) et l'autre fonction polymérisable (double liaison éthylénique) du même monomère greffant est polymérisée avec le polymère souple.

Lorsque le polymère souple ou le polymère de la phase rigide est un polycondensat, on utilise en particulier un polycondensat ayant au moins une insaturation éthylénique apte a réagir avec un monomère comportant également une insaturation éthylénique pour former une liaison covalente avec le polycondensat. Des polycondensats comprenant une ou plusieurs insaturations éthyléniques sont notamment obtenus par polycondensation de monomères tels que l'alcool allylique, la vinylamine, l'acide fumarique. On peut par exemple polymériser des monomères vinyliques avec un polyuréthanne ayant des groupes vinyliques dans ou en extrémité de la chaîne polyuréthanne et greffer ainsi un polymère vinylique sur un polyuréthanne ; une dispersion de particules d'un tel polymère greffé est notamment décrite dans les publications "The structure and properties of acrylic-polyurethane hybrid emulsions", Hiroze M., Progress in Organic Coatings, 38 (2000), pages 27-34 ; "Survey of the applications, properties, and technology of crosslinking emulsions", Bufkin B, Journal of Coatings technology, vol 50, n° 647, Decembre 1978.

Le même principe de greffage s'applique pour les silicones en utilisant des silicones comportant des groupes vinyliques permettant de polymériser sur la silicone des monomères vinyliques et greffer ainsi des chaînes de polymère vinylique sur une silicone.

Les particules à phases rigide et souple ont généralement une taille allant de 1 nm à 10 $\mu$m, en particulier allant de 10 $\mu$m à 1 $\mu$m.

La phase souple peut être présente dans les particules en une teneur d'au moins 1 % en volume, par rapport au volume total de la particule, notamment d'au moins 5 % en volume, en particulier d'au moins 10 % en volume, et plus particulièrement d'au moins 25 % en volume. La phase souple peut être présente dans les particules en une teneur allant jusqu'à 99,999 % en volume, notamment jusqu'à 99,9 % en volume, en particulier jusqu'à 99 % en volume, et plus particulièrement jusqu'à 95 % en volume. En particulier, la phase souple peut être présente dans les particules en une teneur allant de 1 % à 99,999 % en volume, en particulier allant de 5 % à 99,9 % en volume, et plus particulièrement allant de 10 % à 99,9 % en volume, notamment allant de 25 % à 99,9 % en volume, et plus particulièrement allant de 50 % à 95 % en volume, voire allant de 50 % à 90 % en volume.

Dans tous les cas, la phase rigide et la phase souple sont incompatibles, c'est-à-dire qu'elles peuvent être distinguées en utilisant les techniques bien connues de l'homme du métier, telle que par exemple la technique d'observation de microscopie électronique ou la mesure des températures de transitions vitreuses des particules par calorimétrie différentielle. Les particules multiphasées sont donc des particules non homogènes.

La morphologie des phases souple et rigide des particules dispersées peut être, par exemple, de type coeur-écorce, avec des parties d'écorce entourant complètement le coeur, mais aussi coeur-écorce avec une multiplicité de coeurs, ou un réseau interpénétrant de phases. Dans les particules multiphasées, la phase souple est au moins en partie, en particulier en majeure partie, externe, et la phase rigide est au moins en partie, en particulier en majeure partie, interne.

Les particules multiphasées peuvent être préparées par des séries de polymérisation consécutives, avec différents types de monomères. Les particules d'une première famille de monomères sont généralement préparées en une étape séparée, ou formées *in situ* par polymérisation. Ensuite ou en même temps, au moins une autre famille d'autres monomères sont polymérisés au cours d'au moins une étape supplémentaire de polymérisation. Les particules ainsi formées ont au moins une structure au moins en partie interne, ou en noyau, et au moins une structure au moins en partie externe, ou en écorce. La formation d'une structure hétérogène « multicouches » est ainsi possible. Une grande variété de morphologies peut en découler, du type coeur-écorce, mais aussi par exemple avec des inclusions fragmentées de la phase rigide dans la phase souple. Selon l'invention, il est essentiel que la structure en phase souple au moins en partie externe soit plus souple que la structure en phase rigide au moins en partie interne.

Selon un mode de réalisation particulier de l'invention, les particules multiphasées peuvent être dispersées dans un milieu aqueux, notamment un milieu hydrophile. Le milieu aqueux peut être constitué majoritairement d'eau, et en particulier pratiquement totalement d'eau. Ces particules dispersées forment ainsi une dispersion aqueuse de polymère, connue généralement sous le nom de latex ou pseudo-latex. Par « latex », on entend une dispersion aqueuse de particules de polymères telle que l'on peut l'obtenir par polymérisation en émulsion d'au moins un monomère.

La dispersion de particules multiphasées est généralement préparée par au moins une polymérisation en émulsion, en phase continue essentiellement aqueuse, à partir d'initiateurs de réaction, tels que des initiateurs photochimiques ou thermiques pour une polymérisation radicalaire, éventuellement en présence d'additifs tels que des stabilisants, des agents de transfert de chaîne, et/ou des catalyseurs.

Comme dispersions aqueuses de polymère filmogène, on peut utiliser les dispersions acryliques vendues sous les dénominations « Neocryl XK-90® », « Neocryl A-1070® », « Neocryl A-1090® », « Neocryl BT-62® », « Neocryl A-1079® » et « Neocryl A-523® » par la société AVECIA-NEORESINS, « Dow Latex 432® » par la société DOW CHEMICAL, « Daitosol 5000 AD® » ou «Daitosol 5000 SJ » par la société DAITO KASEY KOGYO; « Syntran 5760 » par la société Interpolymer ou encore les dispersions aqueuses de polyuréthanne vendues sous les dénominations « Neorez R-981® » et « Neorez R-974® » par la société AVECIA-NEORESINS, les « Avalure UR-405® », « Avalure UR-410® », « Avalure UR-425® », « Avalure UR-450® », « Sancure 875® », « Sancure 861® », « Sancure 878® » et « Sancure 2060® » par la société GOODRICH, « Impranil 85® » par la société BAYER, « Aquamere H-1511® » par la société HYDROMER ; les sulfopolyesters vendus sous le nom de marque « Eastman AQ® » par la société EASTMAN CHEMICAL PRODUCTS, les dispersions vinyliques comme le « Mexomère PAM », les dispersions aqueuses de polyvinyl acétate comme le « Vinybran® » de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le « Styleze W® »-d'ISP, les dispersions aqueuses de polymère hybrides polyuréthane/polyacryliques telles que celles commercialisées sous les références « Hybridur ® » par la société AIR PRODUCTS ou « Duromer ® » de NATIONAL STARCH, les dispersions de particules de type coeur-écorce telles que celles commercialisées par la société ATOFINA sous la référence « Kynar ® » ( coeur : fluoré -écorce : acrylique) ou encore celles décrites dans US 5 188 899 (coeur : silice - écorce : silicone) et leurs mélanges.

Les dispersions aqueuses de particules utilisées dans l'invention peuvent en outre comprendre différents additifs. Elles peuvent notamment comprendre un agent auxiliaire de filmification favorisant la formation d'un film avec les particules de polymères filmogènes. Un tel agent auxiliaire de filmification peut être choisi parmi tous les composés connus de l'homme du métier comme étant susceptibles de remplir la fonction recherchée et notamment être choisis parmi les agents plastifiants et les agents de coalescence.

## Plastifiant

Selon un mode de réalisation particulier, la dispersion utilisée dans l'invention comprend un agent appelé plastifiant. Cet agent, qui permet de plastifier le polymère en dispersion aqueuse, est généralement un composé organique présentant un coefficient de partage D inférieur ou égal à 0,1. Le coefficient de partage est déterminé conformément à l'enseignement du document publié dans la revue « Progress in Organic Coatings », vol 30, 1997, pp 173-177 intitulé « A method to predict the distribution coefficient of coalescing agents between latex particles and the water phase ».

L'agent plastifiant peut être en particulier choisi parmi l'adipate de diisobutyle, l'ester de l'acide tertio-butylique et du tri-méthyl-2,2,4 pentane-diol-1,3, l'adipate de diéthyle, le phtalate de diéthyle, le phtalate de dibutyle, le phtalate de dioctyle, le phtalate de butyle et de 2-éthyl hexyle, le sébaçate de diméthyle, le sébaçate de dibutyle, le stéarate d'éthyle, le palmitate de 2-éthyl hexyle, le n-butyl éther de dipropylène glycol, et leurs mélanges.

Avantageusement, l'agent plastifiant est choisi parmi l'adipate de diisobutyle, l'ester de l'acide tertio-butylique et du tri-méthyl-2,2,4 pentane-diol-1,3, le n-butyl éther de dipropylène glycol, et leurs mélanges.

L'agent plastifiant peut être présent dans la dispersion utilisée dans l'invention en une teneur allant de 0,1 % à 20 % en poids, et en particulier de 0,5 % à 10 % par rapport au poids total de la dispersion.

Lorsque la dispersion aqueuse de particules est obtenue selon la troisième variante de l'invention, c'est-à-dire lorsque la dispersion aqueuse utilisée comprend un mélange d'au moins deux polymères filmogènes, sous forme de particules solides, distincts par leurs températures de transition vitreuse Tg respectives (Tg1 étant supérieure ou égale à 30 °C et Tg2 étant inférieure ou égale à 0 °C), la dispersion peut être avantageusement exempte ou sensiblement exempte d'agent plastifiant.

## Agent de coalescence

Selon un mode de réalisation particulier, la dispersion utilisée dans l'invention peut comprendre en outre un agent de coalescence. Cet agent qui favorise la coalescence des particules de polymère en dispersion aqueuse, est généralement un solvant organique présentant un coefficient de partage D' supérieur ou égal à 0,5, mesuré selon l'enseignement de la revue « Progress in Organic Coatings », vol 30, 1997, pp 173-177 intitulé « a method to predict the distribution coefficient of coalescing agents between latex particles and the water phase ».

Comme agent de coalescence, on peut utiliser selon invention le n-butyl éther de propylène glycol, le diméthyl éther de dipropylène glycol, l'acétate de méthyl éther de propylène glycol, le propyl éther de propylène glycol, le lactate de méthyle, le lactate d'éthyle, le lactate d'isopropyle, et leurs mélanges.

Avantageusement, l'agent de coalescence est choisi parmi le n-butyl éther de propylène glycol, le diméthyl éther de dipropylène glycol, le lactate d'isopropyle, et leurs mélanges.

L'agent de coalescence peut être présent dans la dispersion en une teneur allant de 0,1 % à 15 % en poids, et en particulier de 0,5 % à 8 % en poids, par rapport au poids total de la dispersion.

Dans un mode de réalisation particulier, le film de la présente invention peut être obtenu par séchage de la dispersion aqueuse ou du mélange de dispersions aqueuses de particules de polymères par chauffage, par exemple à une température pouvant aller de 40 °C et 150 °C. Dans un tel cas, la dispersion peut être avantageusement exempte d'agent de coalescence.

## Phase particulaire additionnelle

La dispersion aqueuse utiliser pour former le film peut comprendre en outre une phase particulaire additionnelle, en une teneur allant de 0,01 à 30 %, notamment de 0,01 à 15 %, en particulier de 0,02 à 10 % et encore mieux de 0,05 à 10 % en poids par rapport au poids total de l'article correspondant. Il peut s'agir d'au moins une matière colorante notamment un pigment et/ou au moins une nacre et/ou au moins des paillettes et/ou au moins une charge complémentaire utilisée dans les compositions cosmétiques.

Par pigments, il faut comprendre des particules blanches ou colorées, minérales ou organiques, destinées à colorer et/ou opacifier le film résultant de la dispersion. Par charges complémentaires, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture du film résultant de la dispersion.

Les pigments peuvent être présents dans l'article à raison de 0,01 à 15 % en poids, notamment de 0,01 à 10 % en poids, et en particulier de 0,02 à 5 % en poids. Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Les nacres peuvent être présentes dans le film résultant à raison de 0,01 à 20 % en poids, de préférence de 0,01 à 15 % en poids, et mieux de 0,02 à 10 % en poids par rapport au poids total du film résultant. Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges complémentaires peuvent être présentes à raison de 0,01 à 20 % en poids, de préférence 0,01 à 15 % en poids, et mieux de 0,02 à 10 % en poids par rapport au poids total de l'article.

On peut notamment citer le talc, le stéarate de zinc, le mica, le kaolin, les poudres de polyamide (Nylon®) (Orgasol® de chez Atochem), les poudres de polyéthylène, les poudres de polymères de tétrafluoroéthyléne (Téflon®), l'amidon, le nitrure de bore, des microsphères polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les organopolysiloxanes élastomères.

La dispersion peut comprendre également des colorants hydrosolubles ou liposolubles en une teneur allant de 0,01 à 10 % en poids, notamment allant de 0,01 à 5 % en poids par rapport au poids total du film résultant. Les colorants liposolubles sont par exemple le rouge Soudan, le DC Red 17, le DC Green 6, le $\beta$-carotène, l'huile de soja, le brun Soudan, le DC Yellow 11, le DC Violet 2, le DC orange 5, le jaune quinoléine. Les colorants hydrosolubles sont par exemple le jus de betterave, le bleu de méthylène.

La dispersion selon l'invention, peut également contenir des ingrédients couramment utilisés en cosmétique et plus spécialement dans le domaine cosmétique et/ou soin des ongles. Ils peuvent notamment être choisis parmi les vitamines, les oligo-éléments, les adoucissants, les séquestrants, les agents alcalinisants ou acidifiants, les agents d'étalement, les agents mouillants, les agents épaississants, les agents dispersants, les anti-mousses, les conservateurs, les filtres UV, les actifs, les agents hydratants, les parfums, les neutralisants, les stabilisants, les antioxydants et leurs mélanges.

Ainsi, lorsque les dispersions selon l'invention sont plus particulièrement destinées au soin des ongles naturels, elles peuvent notamment incorporer, à titre d'actifs, des agents durcissants pour matières kératiniques, des actifs agissant sur la croissance de l'ongle comme par exemple le méthyl sulfonyle méthane et/ou des actifs pour traiter des affections diverses localisées au niveau de l'ongle, comme par exemple l'onichomycose.

Les quantités de ces différents ingrédients sont celles classiquement utilisées dans ce domaine et par exemple de 0,01 à 20 % et notamment de 0,01 à 10 % en poids, par rapport au poids total de l'article.

Selon un mode de réalisation particulier de l'invention, la dispersion aqueuse de polymères peut être avantageusement neutralisée de façon partielle, ce qui limite la quantité de fonctions ionisées. Cela est possible dans la mesure où la stabilité de la dispersion aqueuse n'est pas nécessaire dans le cadre de la présente invention. Ce mode de réalisation de l'invention conduit à un article avantageusement plus résistant à l'eau.

Selon un mode de réalisation particulier de l'invention, la dispersion aqueuse utilisée peut être exempte de gel de laponite ou de gélifiant aqueux habituellement utilisés pour stabiliser les pigments dans des dispersions aqueuses. Un article, conforme à l'invention, réalisé avec une telle dispersion présente une résistance à l'eau accrue.

## MATERIAU ADHESIF

L'article selon l'invention possède une face externe adhésive. Une telle face adhésive est obtenue de façon générale grâce à la présence d'au moins une couche d'au moins un matériau adhésif.

Par « matériau », on entend au sens de la présente invention un polymère ou un système polymérique pouvant comprendre un ou plusieurs polymères de natures différentes. Ce matériau adhésif peut se présenter sous forme d'une solution de polymère ou d'une dispersion de particules de polymères dans un solvant. Ce matériau adhésif peut en outre contenir un plastifiant comme défini ci-dessus. Ce matériau adhésif droit présenter un certain pouvoir collant défini par ses propriétés viscoélastiques.

Les propriétés viscoélastiques d'un matériau sont classiquement définies par deux valeurs caractéristiques qui sont les suivantes :

- le module élastique qui représente le comportement élastique du matériau pour une fréquence donnée et qui est classiquement noté G',
- le module visqueux qui représente le comportement visqueux du matériau pour une fréquence donnée et qui est classiquement noté G".

Ces grandeurs sont notamment définies dans le "Handbook of Pressure Sensitive Adhesive Technology" 3rd edition, D. Satas, chap. 9, p. 155 à 157.

Les matériaux adhésifs utilisables selon la présente invention présentent des propriétés viscoélastiques qui sont mesurées à une température de référence de 35 °C et dans un certain intervalle de fréquences.

Dans le cas de matériaux adhésifs sous forme de solution ou de dispersion de polymère dans un solvant volatil (tel que l'eau, un ester court, un alcool court, de l'acétone ...), on mesure les propriétés viscoélastiques de ce matériau dans des conditions dans lesquelles il présente une teneur en solvant volatil inférieure à 30 %, et en particulier une teneur en solvant volatil inférieure à 20 %.

On mesure en particulier le module élastique du matériau à trois fréquences différentes :

- à faible fréquence, soit à $2.10^{-2}$ Hz,
- à une fréquence intermédiaire, soit à 0,2 Hz,
- à haute fréquence, soit à 2 Hz,

et le module visqueux à la fréquence de 0,2 Hz.

Ces mesures permettent d'évaluer l'évolution du pouvoir collant du matériau adhésif au cours du temps.

Ces propriétés viscoélastiques sont mesurées lors d'essais dynamiques sous sollicitations sinusoïdales de faible amplitude (petites déformations) réalisés à 35 °C sur une plage de fréquence allant de $2.10^{-2}$ à 20 Hz sur un rhéomètre de type "Haake RS50®" sous une sollicitation de torsion/cisaillement, par exemple en géométrie cône-plan (par exemple avec un angle du cône de 1 °).

Avantageusement, ledit matériau adhésif répond aux conditions suivantes :

- G'(2 Hz, 35 °C) $\geq 10^3$ Pa, et
- G'(35 °C.) $\leq 10^8$ Pa, en particulier G'(35 °C) $\leq 10^7$ Pa,
- G'($2.10^{-2}$ Hz, 35 °C) $\leq 3.10^5$ Pa,
  dans lesquelles :

  - G'(2 Hz, 35 °C) est le module élastique de cisaillement dudit matériau adhésif, mesuré à la fréquence de 2 Hz et à la température de 35 °C,
  - G'(35 °C) est le module élastique de cisaillement dudit matériau adhésif, mesuré à la température de 35 °C, pour toute fréquence comprise entre $2.10^{-2}$ et 2 Hz,
  - G'($2.10^{-2}$ Hz, 35 °C) est le module élastique de cisaillement dudit matériau adhésif, mesuré à la fréquence de $2.10^{-2}$ Hz et à la température de 35 °C.

Dans une forme particulière de l'invention, le matériau adhésif répond également à la condition suivante :

- G"/G' (0,2 Hz, 35 °C) $\geq 0,35$.
  dans laquelle :

  - G"(0,2 Hz, 35 °C) est le module visqueux de cisaillement dudit matériau adhésif, mesuré à la fréquence de 0,2 Hz et à la température de 35 °C,
  - G'(0,2 Hz, 35 °C) est le module élastique de cisaillement dudit matériau adhésif, mesuré à la fréquence de 0,2 Hz et à la température de 35 °C.

Dans une forme particulière de l'invention, on a :

- G'(2 Hz, 35 °C) $\geq 5.10^3$ Pa, et en particulier, G'(2 Hz, 35 °C) $\geq 10^4$ Pa. Dans une autre forme particulière de l'invention, on a :
- G'($2.10^{-2}$ Hz, 35 °C) $\leq 5.10^4$ Pa.

En particulier, les matériaux adhésifs selon l'invention répondent aux quatre conditions suivantes :

- G'(2 Hz, 35 °C) $> 10^4$ Pa,
- G'(35 °C.) $\leq 10^8$ Pa, en particulier G'(35 °C) $\leq 10^7$ Pa,
- G'($2.10^{-2}$ Hz, 35 °C) $\leq 5.10^4$ Pa, et
- G"/G'(0,2 Hz, 35 °C. 0,35.

Les matériaux adhésifs selon l'invention peuvent être choisis parmi les adhésifs de type "Pressure Sensitive Adhesives" (adhésifs sensibles à la pression) par exemple, comme ceux cités dans le "Handbook of Pressure Sensitive Adhesive Technology" 3rd édition, D. Satas.

D'une manière générale, la couche adhésive est telle que l'article ne peut pas être ôté par pelage lorsqu'il est appliqué à la surface d'un ongle synthétique ou naturel après au moins 24 heures de pose.

Les matériaux adhésifs selon l'invention sont notamment des polymères choisis parmi les copolymères blocs ou statistiques comprenant au moins un monomère ou une association de monomères dont le polymère résultant à une température de transition vitreuse inférieure à la température ambiante (25 °C), ces monomères ou associations de monomères pouvant être choisis parmi le butadiène, l'éthylène, le propylène, l'isoprène, l'isobutylène, une silicone, et leurs mélanges. Des exemples de tels matériaux sont les polymères blocs de type styrène-butadiène-styrène, styrène-(éthylène-butylène)-styrène, styrène-isoprène-styrène comme ceux vendus sous les dénominations commerciales "Kraton®®" de SHELL CHEMICAL Co. ou de "Vector®" d'EXXON.

Les matériaux adhésifs selon l'invention sont en particulier des polymères adhésifs choisis parmi :

- les polyuréthannes,
- les polymères acryliques,
- les silicones,
- les gommes butyliques, notamment parmi les polyisobutylènes,
- les polymères éthylène-acétate de vinyle,
- les polyamides éventuellement modifiés par des chaînes grasses,
- les gommes naturelles,
- et leur mélanges.

Il peut en particulier s'agir de copolymères adhésifs dérivant de la copolymérisation de monomères vinyliques avec des entités polymériques comme par exemple ceux décrits dans le brevet US 6 136 296. Sont également susceptibles de convenir à l'invention, les copolymères adhésifs décrits dans le brevet US 5 929 173 possédant un squelette polymérique, de Tg variant de 0 °C à 45 °C, greffés par des chaînes dérivant de monomères acryliques et/ou méthacryliques et possédant en revanche une Tg variant de 50 °C à 200 °C.

Les matériaux adhésifs sont par exemple choisis parmi les polyisobutylènes présentant une masse molaire relative Mv supérieure ou égale à 10 000 et inférieure ou égale à 250 000. En particulier, cette masse molaire relative est supérieure ou égale à 18 000 et inférieure ou égale à 150 000.

Comme produits commerciaux convenant particulièrement bien à la présente invention, on peut citer les polyisobutylènes de masses molaires relatives Mv respectives 40 000, 55 000 et 85 000 vendus sous les dénominations commerciales respectives "Oppanol B 10®", "Oppanol B 12®" et "Oppanol B 15®" par la société BASF, et leurs mélanges.

Le matériau adhésif dans l'article conforme à l'invention est généralement sous la forme d'une couche ayant une épaisseur de 1 micron à 100 microns et en particulier de 1 micron à 50 microns, de préférence de 1 micron à 25 microns.

Selon un mode de réalisation particulier de l'invention, la couche formée par le matériau adhésif est directement en contact avec le film polymérique, obtenu par évaporation de la phase aqueuse de la dispersion aqueuse de particules d'au moins un polymère filmogène.

Avantageusement le matériau adhésif et le film présentent une compatibilité grâce à leur nature chimique et leur composition. En effet, dans un mode de réalisation particulier, le solvant de l'adhésif est susceptible de conduire à une augmentation de masse du film de polymère filmogène mis à son contact, notamment d'au moins 10 % en poids par rapport au poids initial du film. En d'autres termes, cette augmentation se traduit par une reprise en masse du film.

Selon une variante de réalisation de l'invention, l'article présente une couche intermédiaire entre la couche de matériau adhésif et le film, obtenu par évaporation de la phase solvant, organique ou aqueuse d'une solution ou dispersion d'au moins un polymère filmogène, colorée ou non. Une telle couche peut être en particulier constituée d'un film de vernis en phase solvant à base de nitrocellulose et/ou au moins d'ester de cellulose notamment coloré. Une telle architecture est particulièrement avantageuse en terme de durée dans le temps. Le film polymérique protège efficacement le film de vernis vis-à-vis des chocs et donc en prolonge significativement la tenue dans le temps.

L'article conforme à la présente invention peut se présenter sous diverses formes telles qu'une étoile, un carré, un rond, etc ....

Comme précisé précédemment, la présente invention couvre également un produit approprié au conditionnement d'un article conforme à la présente invention sous une forme partiellement sèche.

Ce n'est qu'une fois appliqué, que l'article conforme à la présente invention est séché et adopte alors sa structure définitive par mise en contact avec l'air ambiant.

L'article conforme à l'invention présente généralement une épaisseur de 1 micron à 500 microns, notamment de 1 micron à 300 microns et en particulier de 1 micron à 200 microns.

Comme mentionné précédemment, l'article conforme à l'invention est revêtu au moins sur sa face externe adhésive par un support amovible.

Un tel support peut être de toute nature compatible avec le fait que bien qu'il soit en contact avec un matériau adhésif, il peut néanmoins en être séparé.

Le support amovible défini précédemment peut notamment se présenter sous la forme d'une couche protectrice consistant par exemple en un film, en particulier en un film plastique ou un papier ou une structure textile de type feuille.

Avantageusement, ce support est constitué d'un matériau transparent afin de prévenir toute erreur dans le choix de la couleur. Il peut être constitué d'une ou plusieurs couches pouvant être de nature différente. Par exemple, il peut s'agir d'une feuille de papier revêtue avec l'un des plastiques mentionnés ci-après.

Comme film plastique approprié pouvant être par exemple utilisé dans l'article conforme à l'invention, on peut citer des films faits de polyesters, par exemple des téréphtalates de polyéthylène, des téréphtalates de polybutylène ou sébaçates de polyéthylène ou faits de polyéthylène, polypropylène ou polyamides tels que de l'adipate de polyhexaméthylène, du polycaprolactame ou poly(acide oméga-$\omega$-undécanoique amide). En raison de leurs caractéristiques de surface, ces plastiques ne sont bien entendu pas amovibles en tant que tels. Afin de leur conférer cette caractéristique, il est nécessaire de procéder à un traitement de surface à l'aide de substances appropriées telles qu'un traitement par silicones ou, de façon particulièrement avantageuse, par un traitement avec des sels d'acides gras à longues chaînes tels que par exemple en $C_{12}$ à $C_{22}$, ces acides étant saturés ou pouvant contenir jusqu'à trois liaisons oléfines, et au moins des métaux divalents, en particulier des sels de métaux lourds de transition de ce type et en particulier des sels de chrome.

La structure textile de type de feuille peut être un tissé ou non tissé.

Selon un mode de réalisation particulier, l'article conforme à l'invention est revêtu sur ses deux faces avec un support amovible, identique ou différent.

La présente invention concerne également comme indiqué précédemment un procédé de préparation d'un article souple de maquillage et/ou de soin des ongles. Un article conforme à l'invention peut notamment être obtenu avec un dispositif tel que décrit dans le brevet US 4 903 840.

Le procédé selon l'invention comprend une étape d'évaporation de la dispersion aqueuse de particules de polymères filmogènes de façon à obtenir un film. Cette évaporation peut être réalisée selon des méthodes conventionnelles bien connues de l'homme du métier. Elle peut être effectuée par séchage partiel notamment par chauffage, par exemple à une température allant de 20° à 150 °C.

Selon un mode de réalisation particulier du procédé conforme à l'invention, la couche de dispersion aqueuse de particules de polymère filmogène présente une épaisseur pouvant aller de 1 micron à 300 microns, et en particulier de 1 micron à 150 microns.

Le matériau adhésif est généralement déposé sous la forme d'une couche de matériau présentant une épaisseur pouvant aller de 0,5 microns à 200 microns, et en particulier de 1 micron à 100 microns.

Ce procédé peut notamment s'inspirer du procédé décrit dans le brevet US 5 415 903.

L'article obtenu, et en particulier l'excès de film, est ensuite généralement découpé, avant ou après son application, selon la taille et la forme désirée avec de petits ciseaux, un coupe ongles ou en grattant le film.

La présente invention concerne également un procédé de maquillage des ongles dans lequel on applique l'article tel que défini précédemment.

Le maquillage ainsi obtenu peut être éliminé à l'aide des démaquillants usuels dans le domaine des vernis à ongles.

**Revendications**

1. Article souple destiné à être appliqué sur les faux ongles ou les ongles pour leur maquillage et/ou leur soin comprenant au moins :

   - une couche adhésive, et
   - au moins un film polymérique obtenu par évaporation de la phase aqueuse d'une dispersion aqueuse de particules d'au moins un polymère filmogène,

   **caractérisé en ce qu'**il présente à l'état sec une teneur en matière sèche supérieure à 80 % par rapport à son poids total et **en ce qu'**il peut être éliminé par démaquillage à l'aide d'un dissolvant acétone, ester et/ou alcool court.

2. Article selon la revendication 1, **caractérisé en ce qu'**il comprend en outre entre la couche adhésive et le film polymérique au moins un film de vernis coloré.

3. Article selon l'une quelconque des revendications 1 ou 2, **caractérisé en ce que** ledit film polymérique est transparent.

**4.** Article selon la revendication 2 ou 3, **caractérisé en ce que** le film de vernis est obtenu par réticulation ou évaporation de la phase solvant organique ou aqueuse d'une solution ou dispersion d'au moins un polymère filmogène.

**5.** Article selon la revendication 4, **caractérisé en ce que** le film est à base de nitrocellulose et/ou un ester de cellulose.

**6.** Article selon l'une quelconque des revendications précédentes, **caractérisé en ce qu'**il présente une teneur en matière sèche supérieure à 85 % en poids par rapport à son poids total.

**7.** Article selon l'une quelconque des revendications précédentes, **caractérisé en ce qu'**il présente une reprise à l'eau portée à 25 °C inférieure ou égale à 20 %, notamment inférieure ou égale à 16 %, et en particulier inférieure ou égale à 10 %.

**8.** Article selon l'une quelconque des revendications précédentes, **caractérisé en ce qu'**il présente un module de conservation E' supérieur ou égal, à 1 MPa, notamment allant de 1 MPa à 5000 MPa, en particulier supérieur ou égal à 5 MPa, notamment allant de 5 à 1000 MPa, et plus particulièrement supérieur ou égal à 10 MPa par exemple allant de 10 à 500 MPa à une température de 30 °C et une fréquence de 0,1 Hz.

**9.** Article selon l'une quelconque des revendications précédentes, **caractérisé en ce qu'**il possède une déformation à la rupture supérieure ou égale à 5 %, notamment allant de 5 à 500 % et/ou une énergie à rupture par unité de volume $W_r$ supérieure ou égale à 0,2 $J/cm^3$, notamment allant de 0,2 à 100 $J/cm^3$.

**10.** Article selon l'une quelconque des revendications précédentes, **caractérisé en ce que** la taille des particules de polymères de ladite dispersion aqueuse varie de 5 à 500 nm, et est en particulier de 10 à 150 nm.

**11.** Article selon l'une quelconque des revendications précédentes, **caractérisé en ce que** ledit polymère filmogène est choisi parmi les polymères synthétiques de type radicalaire, les polymères synthétiques de type polycondensat et les polymères, d'origine naturelle éventuellement modifiés.

**12.** Article selon la revendication 11, **caractérisé en ce que** ledit polymère synthétique de type radicalaire est un polymère de type homopolymère ou copolymère, vinylique et/ou acrylique.

**13.** Article selon la revendication 11, **caractérise en ce que** ledit polymère synthétique de type polycondensat est choisi parmi les polyuréthannes anioniques, cationiques, non ioniques ou amphotères, les polyuréthannes-acryliques, les polyuréthannes-polyvinylpyrrolidones, les polyesters-polyuréthannes, les polyéther-polyuréthanne, les polyurées, les polyurée/polyurétbannes, les polyuréthannes siliconés, les polyesters, les polyesters amides, les polyesters à chaîne grasse, les polyamides et les résines époxyesters.

**14.** Article selon la revendication 11, **caractérisé en ce que** ledit polymère d'origine naturelle, éventuellement modifié, est choisi parmi la résine shellac, la gomme de sandaraque, les dammars, les élémis, les copals, les polymères cellulosiques insolubles dans l'eau tels que la nitrocellulose et les esters de cellulose modifiés.

**15.** Article selon l'une quelconque des revendications précédentes, **caractérisé en ce que** ladite dispersion aqueuse est une dispersion aqueuse de particules de polyester-polyuréthanne, en particulier anionique.

**16.** Article selon l'une quelconque des revendications précédentes, **caractérisé en ce que** ladite dispersion aqueuse de particules de polymère est une dispersion aqueuse de polymère acrylique, qui présente des propriétés de solubilité à 25 °C dans des solvants organiques correspondant aux paramètres moyens de solubilité dD, dP, et dH de HANSEN satisfaisant aux conditions suivantes :

- dD = 17,5
- dP = 7
- dH = 7,6

avec un rayon R allant de 5 à 10, et en particulier de 5 à 6.

**17.** Article selon l'une quelconque des revendications précédentes, **caractérisé en ce que** ladite dispersion est une dispersion comprenant dans un milieu aqueux acceptable:

a) des particules solides dispersées dans le milieu aqueux d'un premier polymère filmogène ayant au moins une température de transition vitreuse Tg1 supérieure ou égale à 30 °C, et

b) des particules solides dispersées dans le milieu aqueux d'un deuxième polymère filmogène ayant au moins une température de transition vitreuse Tg2 inférieure ou égale à 0 °C,

le premier polymère filmogène étant présent en une teneur allant de 50 % à 90 % en poids, par rapport au poids total des premier et deuxième polymères filmogènes.

**18.** Article selon la revendication 17, **caractérisé en ce que** ladite dispersion aqueuse est une dispersion aqueuse de particules d'au moins un polyuréthanne et d'au moins un polymère radicalaire à groupement carboxylique.

**19.** Article selon l'une quelconque des revendications précédentes, **caractérisé en ce que** ladite dispersion est une dispersion de particules, les particules comprenant au moins une phase souple au moins en partie externe comportant au moins un polymère souple ayant au moins une température de transition vitreuse inférieure ou égal à 60 °C et au moins une phase rigide au moins en partie interne, la phase rigide étant un matériau amorphe ayant au moins une température de transition vitreuse supérieure à 60 °C, le polymère souple étant fixé au moins partiellement par greffage chimique sur la phase rigide.

**20.** Article selon l'une quelconque des revendications précédentes, **caractérisé en ce que** ledit film polymérique est un film multicouche réalisé par superposition d'au moins deux voire plus de couches obtenues respectivement par évaporation de la phase aqueuse de dispersions de particules de polymère(s) filmogène(s) de nature différente.

**21.** Article selon la revendication 20, **caractérisé en ce que** ledit film est réalisé à partir :

- d'une première dispersion aqueuse d'au moins un polymère filmogène ayant au moins une, notamment ayant une, température de transition vitreuse Tg1 inférieure ou égale à 20 °C, notamment allant de -120 °C à 20 °C, et en particulier inférieure à 10 °C, notamment allant de -120 °C à 0 °C, et plus particulièrement allant de -70 °C à -30 °C, ladite première dispersion étant déposée sur un support, puis séchée à une température de 50 à 150 °C, et

- d'une deuxième dispersion aqueuse déposée sur la couche de la première dispersion, ladite deuxième dispersion- aqueuse d'au moins un polymère filmogène ayant au moins une, notamment ayant une, température de transition vitreuse Tg2 supérieure ou égale à 30 °C, notamment allant de 30 °C à 200 °C, et avantageusement supérieure ou égale à 50 °C, notamment allant de 50 °C à 200 °C, et en particulier supérieure ou égale à 80 °C, notamment allant de 80 °C à 180 °C,

l'ensemble étant ensuite séché à une température de 50 à 150 °C, en particulier à une température supérieure à 100 °C.

**22.** Article selon l'une quelconque des revendications précédentes, **caractérisé en ce que** ladite dispersion comprend en outre au moins un agent auxiliaire de filmification.

**23.** Article selon l'une quelconque des revendications précédentes, **caractérisé en ce que** ladite dispersion aqueuse comprend en outre au moins un pigment et/ou au moins une nacre et/ou au moins des paillettes et/ou au moins une charge complémentaire.

**24.** Article selon l'une quelconque des revendications précédentes, **caractérisé en ce que** ladite dispersion comprend en outre un colorant hydrosoluble.

**25.** Article selon l'une quelconque des revendications précédentes, **caractérisé en ce que** ladite couche adhésive comporte au moins un matériau adhésif.

**26.** Article selon l'une quelconque des revendications précédentes, **caractérisé en ce que** la couche adhésive est telle que ledit article ne peut pas être ôté par pelage lorsqu'il est appliqué à la surface d'un ongle synthétique ou naturel après au moins 24 heures de pose.

**27.** Article selon l'une quelconque des revendications 25 ou 26, **caractérisé en ce que** ledit matériau adhésif est choisi parmi les copolymères dérivant de la copolymérisation de monomères vinyliques avec des entités polymériques, des copolymères possédant un squelette polymérique, de Tg variant de 0 °C à 45 °C, greffés par des chaînes dérivant de monomères acryliques et/ou méthacryliques et possédant en revanche une Tg variant de 50 °C à 200 °C et les polyisobutylènes présentant une masse molaire relative Mv supérieure ou égale à 10 000 et inférieure ou égale à 150 000.

**28.** Article selon l'une quelconque des revendications précédents, **caractérisé en ce qu'**il comprend en outre un support amovible, en particulier constitué d'un film plastique modifié par un traitement de surface par silicone ou par des sels d'acides gras en $C_{12}$ à $C_{22}$.

**29.** Produit de maquillage et/ou de soins des ongles et/ou des faux ongles comprenant dans un conditionnement sensiblement étanche à l'air, au moins un article souple destiné à être appliqué sur les faux-ongles ou les ongles pour leur maquillage et/ou leur soin comprenant au moins une couche adhésive, et au moins un film polymérique obtenu par évaporation de la phase aqueuse d'une dispersion aqueuse de particules d'au moins un polymère filmogène, pouvant être démaquillé à l'aide d'un dissolvant, ledit film polymérique pouvant être conforme à l'une quelconque des revendications 3 à 5 et 11 à 24 et ledit article pouvant en outre comprendre les caractéristiques selon les revendications 25 à 28, le conditionnement étant tel que ledit article s'y trouve conservé sous une forme partiellement sèche.

**30.** Produit selon la revendication 29, **caractérisé en ce que** ledit article possède une teneur en matière sèche inférieure à 80 %, en particulier inférieure à 75 % et plus particulièrement inférieure à 70 % en poids par rapport au poids total dudit article.

**31.** Produit selon la revendication, 29 ou 30, **caractérisé en ce que** le conditionnement comporte un réservoir apte à contenir de manière étanche ledit article.

**32.** Procédé de préparation d'un article souple de maquillage et/ou de soin des ongles comprenant au moins les étapes consistant à superposer sur un support amovible:

a) au moins une couche d'une composition à base d'au moins un matériau adhésif, et
b) au moins une couche d'une dispersion aqueuse de particules d'au moins un polymère filmogène, l'évaporation de la phase aqueuse étant réalisée consécutivement au dépôt de celle-ci de manière à obtenir un film polymérique.

**33.** Procédé selon la revendication 32, **caractérisé en ce que** l'on forme en outre, entre la couche adhésive et le film polymérique, un film de vernis coloré.

**34.** Procédé selon la revendication 33, **caractérisé en ce que** le film de vernis est obtenue par réticulation et/ou évaporation de la phase solvant organique ou aqueuse d'une solution ou dispersion d'au moins un polymère filmogène.

**35.** Procédé selon l'une quelconque des revendications 32 à 34, **caractérisé en ce que** ladite dispersion aqueuse de particules de polymère(s) filmogène(s) est telle que définie en revendications 10 à 24.

**36.** Procédé selon l'une quelconque des revendications 32 à 35, **caractérisé en ce que** ledit matériau adhésif est tel que défini en revendications 25 à 27.

**37.** Procédé de préparation d'un produit selon l'une quelconque des revendications 29 à 31, comprenant les étapes consistant à superposer sur un support amovible :

a) au moins une couche d'une composition à base d'au moins un matériau adhésif,
b) au moins une couche d'une dispersion aqueuse de particules d'au moins un polymère filmogène, l'évaporation de la phase aqueuse du film étant effectuée consécutivement au dépôt de ladite composition et contrôlée de manière à obtenir un article partiellement sec, et
c) le conditionnement dudit article à un état partiellement sec au sein d'un conditionnement sensiblement étanche à l'air.

**38.** Procédé de maquillage des ongles utilisant un article tel que défini en revendications 1 à 28, comprenant le fait d'appliquer la face adhésive dudit article sur un ongle naturel ou synthétique.

**Claims**

1. A flexible article for application to false nails or nails to make them up and/or care for them, comprising at least:

   - an adhesive layer; and
   - at least one polymeric film obtained by evaporating the aqueous phase from an aqueous dispersion of particles of at least one film-forming polymer,
   said article being **characterized in that** in the dry state, it has a dry matter content of more than 80% relative to its total weight and **in that** it can be removed by using an acetone, ester and/or lower alcohol remover.

2. An article according to claim 1, **characterized in that** it further comprises at least one film of colored polish between the adhesive layer and the polymeric film.

3. An article according to claim 1 or claim 2, **characterized in that** said polymeric film is transparent.

4. An article according to claim 2 or claim 3, **characterized in that** the film of polish is obtained by cross-linking or evaporating the organic or aqueous solvent phase from a solution or dispersion of at least one film-forming polymer.

5. An article according to claim 4, **characterized in that** the film is based on nitrocellulose and/or a cellulose ester.

6. An article according to any one of the preceding claims, **characterized in that** it has a dry matter content of more than 85% by weight relative to its total weight.

7. An article according to any one of the preceding claims, **characterized in that** it has a water take-up at 25°C of 20% or less, especially 16% or less and in particular 10% or less.

8. An article according to any one of the preceding claims, **characterized in that** it has a storage modulus E' of 1 MPa or more, especially 1 MPa to 5000 MPa, in particular 5 MPa or more, especially 5 to 1000 MPa and more particularly 10 MPa or more, for example 10 MPa to 500 MPa, at a temperature of 30°C and a frequency of 0.1 Hz.

9. An article according to any one of the preceding claims, **characterized in that** it has a deformation at break $\varepsilon_r$ of 5% or more, in particular 5% to 500%, and/or an energy at break per unit volume $W_r$ of 0.2 J/cm$^3$ or more, especially 0.2 J/cm$^3$ to 100 J/cm$^3$.

10. An article according to any one of the preceding claims, **characterized in that** the size of the polymer particles of said aqueous dispersion is from 5 nm to 500 nm, in particular from 10 nm to 150 nm.

11. An article according to any one of the preceding claims, **characterized in that** said film-forming polymer is selected from synthetic radical type polymers, synthetic polycondensate type polymers and polymers of natural origin which may be modified.

12. An article according to claim 11, **characterized in that** said radical type synthetic polymer is a polymer of the homopolymer or copolymer type, vinylic and/or acrylic.

13. An article according to claim 11, **characterized in that** said synthetic polycondensate type polymer is selected from anionic, cationic, non ionic or amphoteric polyurethanes, polyurethane-acrylics, polyurethane-polyvinylpyrrolidones, polyester-polyurethanes, polyether-polyurethanes, polyureas, polyurea/polyurethanes, silicone polyurethanes, polyesters, polyester amides, fatty chain polyesters, polyamides and epoxyester resins.

14. An article according to claim 11, **characterized in that** said polymer of natural origin, which may be modified, is selected from shellac resin, sandarac gum, dammars, elemis, copals, water-insoluble cellulose polymers such as nitrocellulose and modified cellulose esters.

15. An article according to any one of the preceding claims, **characterized in that** said aqueous dispersion is an aqueous dispersion of polyester-polyurethane particles, in particular anionic.

16. An article according to any one of the preceding claims, **characterized in that** said aqueous dispersion of polymer particles is an aqueous dispersion of acrylic polymer which has solubility properties at 25°C in organic solvents corresponding to the mean HANSEN solubility parameters dD, dP and dH satisfying the following conditions:

* dD = 17.5;
* dP = 7;
* dH = 7.6;
with a radius R of 5 to 10, in particular 5 to 6.

17. An article according to any one of the preceding claims, **characterized in that** said dispersion is a dispersion comprising, in an acceptable aqueous medium:

a) solid particles dispersed in the aqueous medium of a first film-forming polymer having at least one glass transition temperature, Tg1, of 30°C or more; and
b) solid particles dispersed in the aqueous medium of a second film-forming polymer having at least one glass transition temperature, Tg2, of 0°C or less;
the first film-forming polymer being present in an amount of 50% to 90% by weight relative to the total weight of the first and second film-forming polymers.

18. An article according to claim 17, **characterized in that** said aqueous dispersion is an aqueous dispersion of particles of at least one polyurethane and at least one radical type polymer with a carboxylic group.

19. An article according to any one of the preceding claims, **characterized in that** said dispersion is a dispersion of particles, the particles comprising at least one flexible phase which is at least partially external, comprising at least one flexible polymer having at least one glass transition temperature of 60°C or less and at least one rigid phase which is at least partially internal, the rigid phase being an amorphous material having at least one glass transition temperature of more than 60°C, the flexible polymer being at least partially fixed by chemical grafting to the rigid phase.

20. An article according to any one of the preceding claims, **characterized in that** said polymeric film is a multi-layered film produced by superposing at least two or more layers respectively obtained by evaporating the aqueous phase from dispersions of particles of film-forming polymer(s) of different natures.

21. An article according to claim 20, **characterized in that** said film is produced from:

- a first aqueous dispersion of at least one film-forming polymer having at least one, especially one glass transition temperature, Tg1, of 20°C or less, especially from -120°C to 20°C, and in particular less than 10°C, especially from -120°C to 0°C, and more particularly from -70°C to -30°C, said first dispersion being disposed on a support then dried at a temperature of 50°C to 150°C; and
- a second aqueous dispersion deposited on the layer of the first dispersion, said second aqueous dispersion of at least one film-forming polymer having at least one, especially one, glass transition temperature, Tg2, of 30°C or more, especially 30°C to 200°C, advantageously 50°C or more, especially 50°C to 200°C, in particular 80°C or more, especially 80°C to 180°C;
the ensemble then being dried at a temperature of 50°C to 150°C, in particular at a temperature of more than 100°C.

22. An article according to any one of the preceding claims, **characterized in that** said dispersion further comprises at least one auxiliary film-forming agent.

23. An article according to any one of the preceding claims, **characterized in that** said aqueous dispersion further comprises at least one pigment and/or at least one nacre and/or at least flakes and/or at least one complementary filler.

24. An article according to any one of the preceding claims, **characterized in that** said dispersion further comprises a hydrosoluble colorant.

25. An article according to any one of the preceding claims, **characterized in that** said adhesive layer comprises at least one adhesive material.

26. An article according to any one of the preceding claims, **characterized in that** the adhesive layer is such that said article cannot be removed by peeling when it is applied to the surface of a synthetic or natural nail after at least 24 hours in position.

**27.** An article according to claim 25 or claim 26, **characterized in that** said adhesive material is selected from copolymers deriving from copolymerizing of vinyl monomers with polymeric entities, copolymers having a polymeric skeleton, with a Tg ranging from 0°C to 45°C, grafted with chains deriving from acrylic and/or methacrylic monomers and having, in contrast, a Tg ranging from 50°C to 200°C, and polyisobutylenes having a relative molar mass Mv of 10000 or more and of 150000 or less.

**28.** An article according to any one of the preceding claims, **characterized in that** it further comprises a removable support, in particular constituted by a plastic film modified by a surface treatment with silicone or with salts of $C_{12}$ to $C_{22}$ fatty acids.

**29.** A product for making up and/or caring for nails and/or false nails comprising, in a packaging which is substantially airtight, at least one flexible article for application to false nails or nails to make them up and/or care for them, comprising at least an adhesive layer, and at least one polymeric film obtained by evaporating the aqueous phase from an aqueous dispersion of particles of at least one film-forming polymer, which article may be removed by using a remover, said polymeric film can be in accordance with any one of claims 3 to 5 and 11 to 24 and said article may further comprise the characteristics according to claims 25 to 28, the packaging being such that said article is preserved in a partially dried form.

**30.** A product according to claim 29, **characterized in that** said article has a dry matter content of less than 80%, in particular less than 75% and more particularly less than 70% by weight relative to the total weight of said article.

**31.** A product according to claim 29 or claim 30, **characterized in that** the packaging comprises a reservoir which can contain said article in a airtight manner.

**32.** A method of preparing a flexible article for making up and/or caring for the nails, comprising at least the steps consisting in superposing on a removable support:

a) at least one layer of a composition based on at least one adhesive material; and
b) at least one layer of an aqueous dispersion of particles of at least one film-forming polymer, the aqueous phase being evaporated off consecutive to deposition thereof to obtain a polymeric film.

**33.** A method according to claim 32, **characterized in that** furthermore, a film of colored varnish is formed between the adhesive layer and the polymeric film.

**34.** A method according to claim 33, **characterized in that** the film of polish is obtained by cross-linking and/or evaporating the organic or aqueous solvent phase from a solution or dispersion of at least one film-forming polymer.

**35.** A method according to any one of claims 32 to 34, **characterized in that** said aqueous dispersion of particles of film-forming polymer(s) is as defined in claims 10 to 24.

**36.** A method according to any one of claims 32 to 35, **characterized in that** said adhesive material is as defined in claims 25 to 27.

**37.** A method of preparing a product according to any one of claims 29 to 31, comprising the steps consisting in superposing on a removable support:

a) at least one layer of a composition based on at least one adhesive material;
b) at least one layer of an aqueous dispersion of particles of at least one film-forming polymer, the aqueous phase being evaporated from the film consecutive to depositing said composition and controlled to obtain a partially dry article; and
c) packaging said article in a partially dry state in substantially airtight packaging.

**38.** A method of making up the nails using an article as defined in claims 1 to 28, comprising applying the adhesive face of said article to a natural or synthetic nail.

**Patentansprüche**

1. Biegsamer Gegenstand, der zum Auftragen auf falsche Nägel oder Nägel, um sie zu schminken und/oder zu pflegen, bestimmt ist, mindestens folgendes umfassend:

   - eine Klebeschicht, und
   - mindestens einen Polymerfilm, erhalten durch Verdunsten der wässrigen Phase einer wässrigen Dispersion von Teilchen mindestens eines filmbildenden Polymers,

   **dadurch gekennzeichnet, dass** er im trockenen Zustand einen Trockensubstanzgehalt von größer als 80 % bezogen auf sein Gesamtgewicht aufweist und dadurch, dass er durch Abschminken mithilfe eines Aceton-, Ester- und/oder kurzkettigen Alkohol-Lösungsmittels entfernt werden kann.

2. Gegenstand nach Anspruch 1, **dadurch gekennzeichnet, dass** er weiterhin zwischen der Klebeschicht und dem Polymerfilm mindestens einen Farblackfilm umfasst.

3. Gegenstand nach einem der Ansprüche 1 oder 2, **dadurch gekennzeichnet, dass** der Polymerfilm transparent ist.

4. Gegenstand nach Anspruch 2 oder 3, **dadurch gekennzeichnet, dass** der Lackfilm durch Vernetzen oder Verdunsten der organischen oder wässrigen Lösungsmittelphase einer Lösung oder Dispersion von mindestens einem filmbildenden Polymer erhalten wird.

5. Gegenstand nach Anspruch 4, **dadurch gekennzeichnet, dass** der Film auf Nitrocellulose und/oder Celluloseester basiert.

6. Gegenstand nach irgendeinem der vorhergehenden Ansprüche, **dadurch gekennzeichnet, dass** er einen Trockensubstanzgehalt von größer als 85 Gew.-% bezogen auf sein Gesamtgewicht aufweist.

7. Gegenstand nach irgendeinem der vorhergehenden Ansprüche, **dadurch gekennzeichnet, dass** er eine Wasserwiederaufnahme bei 25 °C von kleiner oder gleich 20 %, insbesondere von kleiner oder gleich 16 %, und ganz besonders von kleiner oder gleich 10 % aufweist.

8. Gegenstand nach irgendeinem der vorhergehenden Ansprüche, **dadurch gekennzeichnet, dass** er ein Lagerungsmodul E' von größer oder gleich 1 MPa, insbesondere von 1 MPa bis 5.000 MPa, und speziell von größer oder gleich 5 MPa, insbesondere von 5 bis 1.000 MPa, und ganz besonders von größer oder gleich 10 MPa, beispielsweise von 10 bis 500 MPa bei einer Temperatur von 30 °C und einer Frequenz von 0,1 Hz aufweist.

9. Gegenstand nach irgendeinem der vorhergehenden Ansprüche, **dadurch gekennzeichnet, dass** er eine Bruchdehnung $\varepsilon_t$ von größer oder gleich 5 %, insbesondere von 5 bis 500 %, und/oder eine Bruchenergie pro Volumeneinheit $W_r$ von größer oder gleich 0,2 J/cm$^3$, insbesondere von 0,2 bis 100 pro/cm$^3$ aufweist.

10. Gegenstand nach irgendeinem der vorhergehenden Ansprüche, **dadurch gekennzeichnet, dass** die Größe der Polymerteilchen der wässrigen Dispersion von 5 bis 500 nm variiert, und insbesondere von 10 bis 150 nm beträgt.

11. Gegenstand nach irgendeinem der vorhergehenden Ansprüche, **dadurch gekennzeichnet, dass** das filmbildende Polymer aus synthetischen Radikalpolymeren, synthetischen Polykondensat-Polymeren, und aus gegebenenfalls modifizierten Polymeren natürlicher Herkunft ausgewählt ist.

12. Gegenstand nach Anspruch 11, **dadurch gekennzeichnet, dass** das synthetische Radikalpolymer ein Homo- oder Copolymer- eines Vinyl- und/oder Acrylpolymers ist.

13. Gegenstand nach Anspruch 11, **dadurch gekennzeichnet, dass** das synthetische Polykondensat-Polymer ausgewählt ist aus anionischen, kationischen, nichtionischen oder amphoteren Polyurethanen; Polyurethan-Acrylen; Polyurethan-Polyvinylpyrrolidonen; Polyester-Polyurethanen; Polyether-Polyurethanen; Polyharnstoffen; Polyharnstoff/Polyurethanen; silikonierten Polyurethanen; Polyestern; Polyesteramiden; Fettsäurepolyestern; Polyamiden und Epoxidesterharzen.

14. Gegenstand nach Anspruch 11, **dadurch gekennzeichnet, dass** das gegebenenfalls modifizierte Polymer natürlicher Herkunft ausgewählt ist aus Schellack-Harz, Sandarak-Gummi, Dammar, Elemi, Copal, cellulosischen, wasserunlöslichen Polymeren wie Nitrocellulose und modifizierte Celluloseester.

15. Gegenstand nach irgendeinem der vorhergehenden Ansprüche, **dadurch gekennzeichnet, dass** die wässrige Dispersion eine wässrige Dispersion von Teilchen von, insbesondere anionischem, Polyester-Polyurethan ist.

16. Gegenstand nach irgendeinem der vorhergehenden Ansprüche, **dadurch gekennzeichnet, dass** die wässrige Dispersion von Polymerteilchen eine wässrige Dispersion von AcrylPolymer ist, die bei 25 °C in organischen Lösungsmitteln Löslichkeitseigenschaften zeigt, entsprechend den durchschnittlichen. Löslichkeitsparametern dD, dP und dH von HANSEN, die den folgenden Bedingungen genügen:

- dD = 17,5
- dP=7
- dH = 7,6

mit einem Radius R von 5 bis 10, und insbesondere von 5 bis 6.

17. Gegenstand nach irgendeinem der vorhergehenden Ansprüche, **dadurch gekennzeichnet, dass** die Dispersion eine Dispersion ist, die in einem verträglichen wässrigen Medium folgendes umfasst:

a) in dem wässrigen Medium dispergierte feste Teilchen eines ersten filmbildenden Polymers mit mindestens einer Glasübergangstemperatur Tg1 von größer oder gleich 30 °C, und
b) in dem wässrigen Medium dispergierte feste Teilchen eines zweiten filmbildenden Polymers mit mindestens einer Glasübergangstemperatur Tg2 von kleiner oder gleich 0 °C,

wobei das erste filmbildende Polymer in einem Gehalt von 50 Gew.-% bis 90 Gew.-%, bezogen auf das Gesamtgewicht der ersten und zweiten filmbildenden Polymere, vorhanden ist.

18. Gegenstand nach Anspruch 17, **dadurch gekennzeichnet, dass** die wässrige Dispersion eine wässrige Dispersion von Teilchen von mindestens einem Polyurethan und von mindestens einem Radikalpolymer mit Carboxylgruppe ist.

19. Gegenstand nach irgendeinem der vorhergehenden Ansprüche, **dadurch gekennzeichnet, dass** die Dispersion eine Dispersion von Teilchen ist, wobei die Teilchen mindestens eine, mindestens teilweise äußere, biegsame Phase, die mindestens ein biegsames Polymer mit mindestens einer Glasübergangstemperatur von kleiner oder gleich 60°C enthält, und mindestens eine, mindestens teilweise innere, starre Phase umfassen, wobei die starre Phase ein amorphes Material mit mindestens einer Glasübergangstemperatur über 60 °C ist, wobei das biegsame Polymer mindestens zum Teil durch chemische Pfropfung auf der starren Phase angebracht ist.

20. Gegenstand nach irgendeinem der vorhergehenden Ansprüche, **dadurch gekennzeichnet, dass** der Polymerfilm ein mehrschichtiger Film ist, der durch Übereinanderschichten von mindestens zwei, oder sogar mehreren Schichten hergestellt wird, die jeweils durch Verdunsten der wässrigen Phase der Dispersionen von Teilchen von filmbildendem (n) Polymer(en) verschiedener Art erhalten werden.

21. Gegenstand nach Anspruch 20, **dadurch gekennzeichnet, dass** der Film ausgehend von folgendem realisiert wunde:

- einer ersten wässrigen Dispersion mindestens eines filmbildenden Polymers mit mindestens einer, insbesondere einer, Glasübergangstemperatur Tg1 von kleiner oder gleich 20 °C, insbesondere von -120°C bis 20 °C, und speziell von kleiner als 10 °C, insbesondere von -120 °C bis 0 °C, und ganz besonders von -70 °C bis -30 °C, wobei die erste Dispersion auf einem Träger aufgetragen und dann bei einer Temperatur von 50 bis 150 °C getrocknet wird, und
- einer zweiten wässrigen auf der Schicht der ersten Dispersion aufgetragen Dispersion, wobei die zweite wässrige Dispersion mindestens eines filmbildenden Polymers mindestens eine, insbesondere eine, Glasübergangstemperatur Tg2 von größer als oder gleich 30 °C, insbesondere von 30 bis 200 °C und zweckmäßigerweise von grö-βer oder gleich 50 °C., insbesondere von 50 °C bis 200 °C, und speziell von größer oder gleich 80 °C, insbesondere von 80 °C bis 180 °C aufweist,

wobei die Gesamtheit anschließend bei einer Temperatur von 50 bis 150 °C, insbesondere bei einer Temperatur von größer als 100 °C getrocknet wird.

**22.** Gegenstand nach irgendeinem der vorhergehenden Ansprüche, **dadurch gekennzeichnet, dass** die Dispersion weiterhin mindestens ein filmbildendes Hilfsmittel umfasst.

**23.** Gegenstand nach irgendeinem der vorhergehenden Ansprüche, **dadurch gekennzeichnet, dass** die wässrige Dispersion weiterhin mindestens ein Pigment und/oder mindestens einen Perlmuttlack und/oder mindestens Plättchen und/oder mindestens einen ergänzenden Füllstoff umfasst.

**24.** Gegenstand nach irgendeinem der vorhergehenden Ansprüche, **dadurch gekennzeichnet, dass** die Dispersion weiterhin ein wasserlösliches Farbmittel umfasst.

**25.** Gegenstand nach irgendeinem der vorhergehenden Ansprüche, **dadurch gekennzeichnet, dass** die Klebeschicht mindestens ein Klebematerial umfasst.

**26.** Gegenstand nach irgendeinem der vorhergehenden Ansprüche, **dadurch gekennzeichnet, dass** die Klebeschicht so beschaffen ist, dass der Gegenstand nach mindestens 24 Stunden Ruhe nicht durch Abziehen abgelöst werden kann, wenn er auf die Oberfläche eines Kunstnagels oder eines Naturnagels aufgebracht wurde.

**27.** Gegenstand nach irgendeinem der Ansprüche 25 oder 26, **dadurch gekennzeichnet, dass** das Klebematerial ausgewählt ist aus Copolymeren, die sich aus der Copolymerisation von Vinyl-Monomeren mit Polymereinheiten ableiten, Copolymeren, die ein Polymerskelett besitzen, mit einer Tg, die von 0 °C bis 45 °C variiert, gepropft mit Ketten, die sich von Acryl-Monomeren und/oder Methacryl-Monomeren ableiten und die wiederum eine Tg besitzen, die von 50 °C bis 200 °C variiert, und Polyisobutylenen, die eine relative Molmasse Mv von größer oder gleich 10.000 und von kleiner oder gleich 150.000 aufweisen.

**28.** Gegenstand nach irgendeinem der vorhergehenden Ansprüche, **dadurch gekennzeichnet, dass** er weiterhin einen abnehmbaren Träger umfasst, insbesondere bestehend aus einem Kunststofffilm, der durch eine Oberflächenbehandlung mit Silikon oder durch Salze von $C_{12}$- bis $C_{22}$-Fettsäuren modifiziert ist.

**29.** Schminkprodukt und/oder Pflegeprodukt für Nägel und/oder für falsche Nägel, umfassend, in einer im wesentlichen luftdichte Verpackung, mindestens einen biegsamen Gegenstand, der dazu bestimmt ist, auf die falschen Nägel oder die Nägel zu deren Schminken und/oder zu deren Pflege aufgebracht zu werden, umfassend mindestens eine Klebeschicht und mindestens einen Polymerfilm, der durch Verdunsten der wässrigen Phase einer wässrigen Dispersion von Teilchen mindestens eines filmbildenden Polymers erhalten wird, der mithilfe eines Lösungsmittels abgeschminkt werden kann, wobei der Polymerfilm gemäß irgendeinem der Ansprüche 3 bis 5 und 11 bis 24 sein kann, und der Gegenstand weiterhin die Merkmale gemäß der Ansprüche 25 bis 28 einschließen kann, wobei die Verpackung dergestalt ist, dass der Gegenstand in einer teilweise getrockneten Form konserviert vorliegt.

**30.** Produkt nach Anspruch 29, **dadurch gekennzeichnet, dass** der Gegenstand einen Trockensubstanzgehalt von kleiner als 80 %, insbesondere von kleiner als 75 %, und ganz besonders kleiner als 70 Gew.-%, bezogen auf das Gesamtgewicht des Gegenstandes umfasst.

**31.** Produkt nach Anspruch 29 oder 30, **dadurch gekennzeichnet, dass** die Verpackung ein Reservoir umfasst, das dazu geeignet ist, den Gegenstand dicht zu enthalten.

**32.** Verfahren zur Herstellung eines biegsamen Gegenstands zum Schminken und/oder zur Pflege der Nägel, umfassend mindestens die Schritte, bestehend aus der Übereinanderlagerung von folgendem auf einem abnehmbaren Träger:

a) mindestens einer Schicht einer Zusammensetzung auf der Grundlage mindestens eines Klebematerials, und
b) mindestens einer Schicht einer wässrigen Dispersion von Teilchen mindestens eines filmbildenden Polymers, wobei die Verdunstung der wässrigen Phase nach ihrem Auftragen vorgenommen wird, so dass ein Polymerfilm erhalten wird.

**33.** Verfahren nach Anspruch 32, **dadurch gekennzeichnet, dass** weiterhin zwischen der Klebeschicht und dem Polymerfilm ein Farblackfilm ausgebildet wird.

**34.** Verfahren nach Anspruch 33, **dadurch gekennzeichnet, dass** der Lackfilm durch Vernetzen und/oder Verdunsten der organischen oder wässrigen Lösungsmittelphase der Lösung oder Dispersion mindestens eines filmbildenden Polymers erhalten wird.

35. Verfahren nach einem der Ansprüche 32 bis 34, **dadurch gekennzeichnet, dass** die wässrige Dispersion von Teilchen von filmbildendem(n) Polymer(en) so beschaffen ist wie in den Ansprüchen 10 bis 24 definiert.

36. Verfahren nach einem der Ansprüche 32 bis 35, **dadurch gekennzeichnet, dass** das Klebematerial so beschaffen ist wie in den Ansprüchen 25 bis 27 definiert.

37. Verfahren zur Herstellung eines Produkts gemäß einem der Ansprüche 29 bis 31, umfassend die Schritte des Übereinanderschichtens von folgendem auf einem abnehmbaren Träger:

   a) mindestens einer Schicht aus einer Zusammensetzung auf der Grundlage von mindestens einem Klebematerial,
   b) mindestens einer Schicht einer wässrigen Dispersion von Teilchen mindestens eines filmbildenden Polymers, wobei das Verdunsten der wässrigen Phase des Films nach dem Auftragen der Zusammensetzung durchgeführt und so gesteuert wird, dass ein teilweise trockener Gegenstand erhalten wird, und
   c) der Verpackung des Gegenstandes in einem teilweise trockenen Zustand in einer im wesentlichen luftdichten Verpackung.

38. Schminkverfahren der Nägel unter Verwendung eines Gegenstands wie in den Ansprüchen 1 bis 28 definiert, umfassend das Aufbringen der klebrigen Seite des Gegenstandes auf einem Natur- oder Kunstnagel.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2803743 A1
- US 5747018 A
- US 2003185774 A
- US 5188899 A
- EP 542669 A
- EP 787730 A
- EP 787731 A
- WO 9608537 A
- US 6136296 A
- US 5929173 A
- US 4903840 A
- US 5415903 A

**Littérature non-brevet citée dans la description**

- **ALLAN F. M. BARTON.** CRC Handbook of solubility parameters and other cohesion parameters. 1991, 95-109
- **HIROZE M.** The structure and properties of acrylic-polyurethane hybrid emulsions. *Progress in Organic Coatings,* 2000, vol. 38, 27-34
- **BUFKIN B.** Survey of the applications, properties, and technology of crosslinking emulsions. *Journal of Coatings technology,* Décembre 1978, vol. 50 (647
- A method to predict the distribution coefficient of coalescing agents between latex particles and the water phase. *Progress in Organic Coatings,* 1997, vol. 30, 173-177
- a method to predict the distribution coefficient of coalescing agents between latex particles and the water phase. *Progress in Organic Coatings,* 1997, vol. 30, 173-177
- Handbook of Pressure Sensitive Adhesive Technology. 155-157
- Handbook of Pressure Sensitive Adhesive Technology